# EUROPEAN PATENT SPECIFICATION

(11) **EP 2 875 136 B1**
(45) Date of publication and mention of the grant of the patent: **12.04.2017**
(21) Application number: 13770715.4
(22) Date of filing: 18.07.2013
(51) Int. Cl.: C12N 15/74, C12P 5/00

(54) **RHODOBACTER FOR PREPARING TERPENOIDS**
RHODOBACTER ZUR HERSTELLUNG VON TERPENOIDEN
RHODOBACTER POUR PRÉPARER DES TERPÉNOÏDES

(30) Priority: 18.07.2012 EP 12176922
(43) Date of publication of application: 27.05.2015
(73) Proprietor: Isobionics B.V., 6167 RD Geleen (NL)
(72) Inventor: HUEMBELIN, Markus, 4058 Basel (CH); BEEKWILDER, Martinus Julius, 6871 WG Renkum (NL); KIERKELS, Johannes Gerardus Theodorus, 6133 BH Sittard (NL)
(74) Representative: Wulferink, Marty Bernardus Franciscus
(86) International application number: PCT/NL2013/000040
(87) International publication number: WO 2014/014339

(56) References cited:
- EP-A1- 2 336 310
- WO-A1-2006/018211
- WO-A2-2009/042070
- WO-A2-2009/064910
- WO-A2-2012/177129

## Description

The invention relates to a *Rhodobacter* host cell, comprising a nucleic acid encoding an enzyme having catalytic activity in the synthesis of a monoterpene. The invention further relates to the use of a *Rhodobacter* host cell in the production of a monoterpene and to a method for preparing a monoterpene.

Many organisms have the capacity to produce a wide array of terpenes and terpenoids. Terpenes are actually or conceptually built up from 2-methylbutane residues, usually referred to as units of isoprene, which has the molecular formula C₅H₈. One can consider the isoprene unit as one of nature's common building blocks. The basic molecular formulae of terpenes are multiples of that formula: (C₅H₈)ₙ, wherein n is the number of linked isoprene units. This is called the isoprene rule, as a result of which terpenes are also denoted as isoprenoids. The isoprene units may be linked together "head to tail" to form linear chains or they may be arranged to form rings. In their biosynthesis, terpenes are formed from the universal 5 carbon precursors isopentenyl diphosphate (IPP) and its isomer, dimethylallyl diphosphate (DMAPP). Accordingly, a terpene carbon skeleton generally comprises a multiple of 5 carbon atoms. Most common are the 5-, 10-, 15-, 20-, 30- and 40-carbon terpenes, which are referred to as hemi-, mono-, sesqui-, di-, tri- and tetraterpenes, respectively. Besides "head-to-tail" connections, tri- and tetraterpenes also contain one "tail-to-tail" connection in their centre. The terpenes may comprise further functional groups, like alcohols and their glycosides, ethers, aldehydes, ketones, carboxylic acids and esters. These functionalised terpenes are herein referred to as terpenoids. Like terpenes, terpenoids generally have a carbon skeleton having a multiple of 5 carbon atoms. It should be noted that the total number of carbons in a terpenoid does not need to be a multiple of 5, *e.g.* the functional group may be an ester group comprising an alkyl group having any number of carbon atoms.

Apart from the definitions given above, it is important to note that the terms "terpene", "terpenoid" and "isoprenoid" are frequently used interchangeably in the art.

Terpenoids are amongst others industrially applicable as an aroma or flavour. They may also serve as intermediate compounds for other industrially applicable compounds, *e*.*g*. other flavour compounds or aroma compounds.

Traditionally, terpenoids, such as monoterpenes and sesquiterpenes have been obtained by extraction from natural sources. However, the yield of extraction methods is usually low. Also, a suitable extraction technique may require the use of toxic solvents, whereby special handling and disposal procedures are needed. Further, the composition of the crude extract may vary, depending on the batch of the source material. This may result in varying product properties or may necessitate variations in the purification process of the desired terpenoid from the crude extract. Valencene is an example of a sesquiterpene produced in specific plants, such as various citrus fruits. Valencene can be obtained by distillation from citrus essential oils obtained from citrus fruits, but isolation from these oils is cumbersome because of the low valencene concentration in these fruits (0.2 to 0.6% by weight).

Beta-pinene is an example of a monoterpene. Natural sources of beta-pinene include pine trees, rosemary, parsley, dill, basil and rose.

It has been proposed to prepare terpenoids microbiologically, making use of micro-organisms genetically modified by incorporation of a gene that is coding for a protein having terpenoid synthase activity, in addition to genes encoding enzymes for production of IPP, either via the mevalonate pathway or the DXP pathway. These pathways are known in the art, and have been described, e.g., by Withers & Keasling in Appl. Microbiol. Biotechnol. (2007) 73: 980-990, in particular in Figure 1.

According to US 7,659,097, there remains a need for expression systems and fermentation procedures that produce even more isoprenoids than available with current technologies. Further, optimal redirection of microbial metabolism toward isoprenoid production requires that the introduced biosynthetic pathway is properly engineered both to funnel carbon to isoprenoid production efficiently and to prevent build up of toxic levels of metabolic intermediates over a sustained period of time. In order to accomplish this, it is proposed to produce an isoprenoid in a method making use of a bacterial or fungal host cell in which heterologous enzymes of the mevalonate pathway or of the DXP pathway are expressed and wherein the cells are grown in a carbon limited medium, reducing the growth to 75% or less of the maximum specific growth rate.

It is an object of the present invention to provide a novel host cell that can be used as an alternative to known host cells for producing a monoterpene; in particular it is an object to provide a method or a host cell for a method for producing a monoterpene with good yield, with high specificity, with good productivity (in particular good specific productivity) or a low tendency to build op toxic levels of metabolic intermediates over a sustained period of time.

The inventors have realised that a cell of the genus *Rhodobacter* is an organism that is particularly interesting as a host cell suitable for genetic modification into a host cell that can be used for the production of a monoterpene.

Accordingly, the present invention relates to a *Rhodobacter* host cell, comprising a nucleic acid encoding
- enzymes of a mevalonate pathway for making isoprenyl pyrophosphate (IPP) and its isomer dimethylallyl pyrophosphate (DMAPP);
- an enzyme having catalytic activity for the condensation of IPP and DMAPP into geranyl diphosphate (GPP) and
- an enzyme having monoterpene synthase activity in the conversion of GPP into a monoterpene, wherein the enzyme having monoterpene synthase activity is a beta-pinene synthase comprising a sequence having at least 40 % sequence identity with SEQ ID NO: 2.

Generally, said *Rhodobacter* host cell comprises one or more homologous genes encoding an enzyme having catalytic activity in the reaction of conversion of two molecules of acetyl-CoA to acetoacyl-CoA. Preferably, said *Rhodobacter* host cell is free of heterologous enzymes having catalytic activity in the reaction of conversion of two molecules of acetyl-CoA to acetoacyl-CoA.

The invention further relates to the use of a *Rhodobacter* host cell according to the invention in the production of a monoterpene, preferably a monoterpene selected from the group of beta-pinene, myrcene, alpha-pinene, limonene, sabinene, and geraniol.

The invention further relates to a method for preparing a monoterpene, comprising culturing a host cell according to the invention in a culture medium comprising a carbon source (for instance a sugar) for the monoterpene.

It is the inventors insight that it is surprisingly possible to produce a terpenoid in a bacterial host cell, also in the absence of a heterologous acetyl-CoA tholase. This is in particular surprising, since heterologous expression of the mevalonate pathway in other bacterial hosts, in particular in E. coli, was described to include a heterologously expressed thiolase (*e*.*g*. claim 1 in US 7,172,886 and example 1 in US 7,659,097).

In an advantageous embodiment, the host cell has an improved productivity, compared to a known microbiological method for producing a monoterpene of interest. As used herein 'productivity', is defined as the molar amount of reaction product, *i.e.* monoterpene of interest, such as beta-pinene, formed in a suitable culture medium, per unit of time. Standard conditions can be based on, *e*.*g*., WO2011/074954 page 68 (examples, general part, shake-flask procedure).

The term "or" as used herein is defined as "and/or" unless specified otherwise.

The term "a" or "an" as used herein is defined as "at least one" unless specified otherwise.

When referring to a noun (*e*.*g*. a compound, an additive, etc.) in the singular, the plural is meant to be included.

The terms farnesyl diphosphate and farnesyl pyrophosphate (both abbreviated as FPP) as interchangeably used herein refer to the compound 3,7,11-trimethyl-2,6,10-dodecatrien-1-yl pyrophosphate and include all known isomers of this compound.

The term "recombinant" in relation to a recombinant cell, vector, nucleic acid or the like as used herein, refers to a cell, vector, nucleic acid or the like, containing nucleic acid not naturally occurring in that cell, vector, nucleic acid or the like and/or not naturally occurring at that same location. Generally, said nucleic acid has been introduced into that strain (cell) using recombinant DNA techniques.

The term "heterologous" when used with respect to a nucleic acid (DNA or RNA) or protein refers to a nucleic acid or protein that does not occur naturally as part of the organism, cell, genome or DNA or RNA sequence in which it is present, or that is found in a cell or location or locations in the genome or DNA or RNA sequence that differ from that in which it is found in nature. Heterologous nucleic acids or proteins are not endogenous to the cell into which they are introduced, but have been obtained from another cell or synthetically or recombinantly produced. Generally, though not necessarily, such nucleic acids encode proteins that are not normally produced by the cell in which the DNA is expressed.

A gene that is endogenous to a particular host cell but has been modified from its natural form, through, for example, the use of DNA shuffling, is also called heterologous. The term "heterologous" also includes non-naturally occurring multiple copies of a naturally occurring DNA sequence. Thus, the term "heterologous" may refer to a DNA segment that is foreign or heterologous to the cell, or homologous to the cell but in a position and/or a number within the host cell nucleic acid in which the segment is not ordinarily found. Exogenous DNA segments are expressed to yield exogenous polypeptides. A "homologous" DNA sequence is a DNA sequence that is naturally associated with a host cell into which it is introduced.

Any nucleic acid or protein that one of skill in the art would recognize as heterologous or foreign to the cell in which it is expressed is herein encompassed by the term heterologous nucleic acid or protein.

The terms "modified", "modification", "mutated", "mutation", or "variant" as used herein regarding proteins or polypeptides compared to another protein or peptide (in particular compared to the polypeptide consisting of amino acids in the sequences shown herein), is used to indicate that the modified protein or polypeptide has at least one difference in the amino acid sequence compared to the protein or polypeptide with which it is compared, *e*.*g*. a wild-type protein/polypeptide. The terms are used irrespective of whether the modified/mutated protein actually has been obtained by mutagenesis of nucleic acids encoding these amino acids or modification of the polypeptide/protein or in another manner, *e*.*g*. using artificial gene-synthesis methodology. Mutagenesis is a well-known method in the art, and includes, for example, site-directed mutagenesis by means of PCR or via oligonucleotide-mediated mutagenesis as described in Sambrook, J., and Russell, D.W. Molecular Cloning: A Laboratory Manual.3d ed., Cold Spring Harbor Laboratory Press, Cold Spring Harbor, NY, (2001). The term "modified", "modification", "mutated", "mutation" or "variant" as used herein regarding genes is used to indicate that at least one nucleotide in the nucleotide sequence of that gene or a regulatory sequence thereof, is different from the nucleotide sequence that it is compared with, *e*.*g*. a wild-type nucleotide sequence. The terms are used irrespective of whether the modified/mutated nucleotide sequence actually has been obtained by mutagenesis.

A modification/mutation may in a particular be a replacement of an amino acid respectively nucleotide by a different one, a deletion of an amino acid respectively nucleotide or an insertion of an amino acid respectively nucleotide.

The terms "open reading frame" and "ORF" refer to the amino acid sequence encoded between translation initiation and termination codons of a coding sequence. The terms "initiation codon" and "termination codon" refer to a unit of three adjacent nucleotides ('codon') in a coding sequence that specifies initiation and chain termination, respectively, of protein synthesis (mRNA translation).

The term "gene" is used broadly to refer to any segment of nucleic acid associated with a biological function. Thus, genes include coding sequences and/or the regulatory sequences required for their expression. For example, gene refers to a nucleic acid fragment that expresses mRNA or functional RNA, or encodes a specific protein, and which includes regulatory sequences. Genes also include non-expressed DNA segments that, for example, form recognition sequences for other proteins. Genes can be obtained from a variety of sources, including cloning from a source of interest or synthesizing from known or predicted sequence information, and may include sequences designed to have desired parameters.

The term "chimeric gene" refers to any gene that contains 1) DNA sequences, including regulatory and coding sequences that are not found together in nature, or 2) sequences encoding parts of proteins not naturally adjoined, or 3) parts of promoters that are not naturally adjoined. Accordingly, a chimeric gene may comprise regulatory sequences and coding sequences that are derived from different sources, or comprise regulatory sequences and coding sequences derived from the same source, but arranged in a manner different from that found in nature.

The term "transgenic" for a transgenic cell or organism as used herein, refers to an organism or cell (which cell may be an organism *per se* or a cell of a multicellular organism from which it has been isolated) containing a nucleic acid not naturally occurring in that organism or cell and which nucleic acid has been introduced into that organism or cell (*i.e.* has been introduced in the organism or cell itself or in an ancestor of the organism or an ancestral organism of an organism of which the cell has been isolated) using recombinant DNA techniques.

A "transgene" refers to a gene that has been introduced into the genome by transformation and preferably is stably maintained. Transgenes may include, for example, genes that are either heterologous or homologous to the genes of a particular cell/organism to be transformed. Additionally, transgenes may comprise native genes inserted into a non-native organism, or chimeric genes. The term "endogenous gene" refers to a native gene in its natural location in the genome of an organism. A "foreign" gene refers to a gene not normally found in the host organism but that is introduced by gene transfer.

"Transformation" and "transforming", as used herein, refers to the introduction of a heterologous nucleotide sequence into a host cell, irrespective of the method used for the insertion, for example, direct uptake, transduction, conjugation, f-mating or electroporation. The exogenous polynucleotide may be maintained as a non-integrated vector, for example, a plasmid, or alternatively, may be integrated into the host cell genome.

"Coding sequence" refers to a DNA or RNA sequence that codes for a specific amino acid sequence and excludes the non-coding sequences. It may constitute an "uninterrupted coding sequence", *i.e.* lacking an intron, such as in a cDNA or it may include one or more introns bound by appropriate splice junctions. An "intron" is a sequence of RNA which is contained in the primary transcript but which is removed through cleavage and re-ligation of the RNA within the cell to create the mature mRNA that can be translated into a protein.

"Regulatory sequences" refer to nucleotide sequences located upstream (5' non-coding sequences), within, or downstream (3' non-coding sequences) of a coding sequence, and which influence the transcription, RNA processing or stability, or translation of the associated coding sequence. Regulatory sequences include enhancers, promoters, translation leader sequences, introns, and polyadenylation signal sequences. They include natural and synthetic sequences as well as sequences which may be a combination of synthetic and natural sequences. As is noted above, the term "suitable regulatory sequences" is not limited to promoters.

Examples of regulatory sequences include promoters (such as transcriptional promoters, constitutive promoters, inducible promoters), operators, enhancers, mRNA ribosomal binding sites, and appropriate sequences which control transcription and translation initiation and termination. Nucleic acid sequences are "operably linked" when the regulatory sequence functionally relates to the DNA or cDNA sequence. As used herein, the term "operably linked" or "operatively linked" refers to a juxtaposition wherein the components so described are in a relationship permitting them to function in their intended manner. A control sequence "operably linked" to another control sequence and/or to a coding sequence is ligated in such a way that transcription and/or expression of the coding sequence is achieved under conditions compatible with the control sequence. Generally, operably linked means that the nucleic acid sequences being linked are contiguous and, where necessary to join two protein coding regions, contiguous and in the same reading frame.

Each of the regulatory sequences may independently be selected from heterologous and homologous regulatory sequences.

"Promoter" refers to a nucleotide sequence, usually upstream (5') to its coding sequence, which controls the expression of said coding sequence by providing the recognition for RNA polymerase and other factors required for proper transcription. "Promoter" includes a minimal promoter that is a short DNA sequence comprised of a TATA box and other sequences that serve to specify the site of transcription initiation, to which regulatory elements are added for control of expression. "Promoter" also refers to a nucleotide sequence that includes a minimal promoter plus regulatory elements that is capable of controlling the expression of a coding sequence or functional RNA. This type of promoter sequence consists of proximal and more distal upstream elements, the latter elements often referred to as enhancers. Accordingly, an "enhancer" is a DNA sequence which can stimulate promoter activity and may be an innate element of the promoter or a heterologous element inserted to enhance the level or tissue specificity of a promoter. It is capable of operating in both orientations (normal or flipped), and is capable of functioning even when moved either upstream or downstream from the promoter. Both enhancers and other upstream promoter elements bind sequence-specific DNA-binding proteins that mediate their effects. Promoters may be derived in their entirety from a native gene, or be composed of different elements derived from different promoters found in nature, or even be comprised of synthetic DNA segments. A promoter may also contain DNA sequences that are involved in the binding of protein factors which control the effectiveness of transcription initiation in response to physiological or developmental conditions.

The term "nucleic acid" as used herein, includes reference to a deoxyribonucleotide or ribonucleotide polymer, *i.e.* a polynucleotide, in either single-or double-stranded form, and unless otherwise limited, encompasses known analogues having the essential nature of natural nucleotides in that they hybridize to single-stranded nucleic acids in a manner similar to naturally occurring nucleotides (*e*.*g*., peptide nucleic acids). A polynucleotide can be full-length or a subsequence of a native or heterologous structural or regulatory gene. Unless otherwise indicated, the term includes reference to the specified sequence as well as the complementary sequence thereof. Thus, DNAs or RNAs with backbones modified for stability or for other reasons are "polynucleotides" as that term is intended herein. Moreover, DNAs or RNAs comprising unusual bases, such as inosine, or modified bases, such as tritylated bases, to name just two examples, are "polynucleotides" as the term is used herein. It will be appreciated that a great variety of modifications have been made to DNA and RNA that serve many useful purposes known to those of skill in the art. The term "polynucleotide" as it is employed herein embraces such chemically, enzymatically or metabolically modified forms of polynucleotides, as well as the chemical forms of DNA and RNA characteristic of viruses and cells, including among other things, simple and complex cells.

Every nucleic acid sequence herein that encodes a polypeptide also, by reference to the genetic code, describes every possible silent variation of the nucleic acid. The term "conservatively modified variants" applies to both amino acid and nucleic acid sequences. With respect to particular nucleic acid sequences, the term "conservatively modified variants" refers to those nucleic acids which encode identical or conservatively modified variants of the amino acid sequences due to the degeneracy of the genetic code. The term "degeneracy of the genetic code" refers to the fact that a large number of functionally identical nucleic acids encode any given protein. For instance, the codons GCA, GCC, GCG and GCU all encode the amino acid alanine. Thus, at every position where an alanine is specified by a codon, the codon can be altered to any of the corresponding codons described without altering the encoded polypeptide. Such nucleic acid variations are "silent variations" and represent one species of conservatively modified variation. The terms "polypeptide", "peptide" and "protein" are used interchangeably herein to refer to a polymer of amino acid residues. The terms apply to amino acid polymers in which one or more amino acid residue is an artificial chemical analogue of a corresponding naturally occurring amino acid, as well as to naturally occurring amino acid polymers. The essential nature of such analogues of naturally occurring amino acids is that, when incorporated into a protein, that protein is specifically reactive to antibodies elicited to the same protein but consisting entirely of naturally occurring amino acids. The terms "polypeptide", "peptide" and "protein" are also inclusive of modifications including, but not limited to, glycosylation, lipid attachment, sulphation, gamma-carboxylation of glutamic acid residues, hydroxylation and ADP-ribosylation.

Within the context of the present application, oligomers (such as oligonucleotides, oligopeptides) are considered a species of the group of polymers. Oligomers have a relatively low number of monomeric units, in general 2-100, in particular 6-100.

"Expression cassette" as used herein means a DNA sequence capable of directing expression of a particular nucleotide sequence in an appropriate host cell, comprising a promoter operably linked to the nucleotide sequence of interest which is operably linked to termination signals. It also typically comprises sequences required for proper translation of the nucleotide sequence. The coding region usually codes for a protein of interest but may also code for a functional RNA of interest, for example antisense RNA or a non-translated RNA, in the sense or antisense direction. The expression cassette comprising the nucleotide sequence of interest may be chimeric, meaning that at least one of its components is heterologous with respect to at least one of its other components. The expression cassette may also be one which is naturally occurring but has been obtained in a recombinant form useful for heterologous expression. The expression of the nucleotide sequence in the expression cassette may be under the control of a constitutive promoter or of an inducible promoter which initiates transcription only when the host cell is exposed to some particular external stimulus. In the case of a multicellular organism, the promoter can also be specific to a particular tissue or organ or stage of development.

The term "vector" as used herein refers to a construction comprised of genetic material designed to direct transformation of a targeted cell. A vector contains multiple genetic elements positionally and sequentially oriented, *i*.*e*., operatively linked with other necessary elements such that the nucleic acid in a nucleic acid cassette can be transcribed and when necessary, translated in the transformed cells.

In particular, the vector may be selected from the group of viral vectors, (bacterio)phages, cosmids or plasmids. The vector may also be a yeast artificial chromosome (YAC), a bacterial artificial chromosome (BAC) or *Agrobacterium* binary vector. The vector may be in double or single stranded linear or circular form which may or may not be self transmissible or mobilizable, and which can transform *Rhodobacter* host organisms either by integration into the cellular genome or exist extrachromosomally (*e*.*g*. autonomous replicating plasmid with an origin of replication). Specifically included are shuttle vectors by which is meant a DNA vehicle capable, naturally or by design, of replication in two different host organisms, which may be selected from actinomycetes and related species, bacteria and eukaryotic (*e*.*g*. higher plant, mammalian, yeast or fungal) cells. Preferably the nucleic acid in the vector is under the control of, and operably linked to, an appropriate promoter or other regulatory elements for transcription in a host cell such as a microbial, *e*.*g*. bacterial, or plant cell. The vector may be a bi-functional expression vector which functions in multiple hosts. In the case of genomic DNA, this may contain its own promoter or other regulatory elements and in the case of cDNA this may be under the control of an appropriate promoter or other regulatory elements for expression in the host cell.

Vectors containing a nucleic acid can be prepared based on methodology known in the art *per se.* For instance use can be made of a cDNA sequence encoding the polypeptide operably linked to suitable regulatory elements, such as transcriptional or translational regulatory nucleic acid sequences.

The term "vector" as used herein, includes reference to a vector for standard cloning work ("cloning vector") as well as to more specialized type of vectors, like an (autosomal) expression vector and a cloning vector used for integration into the chromosome of the host cell ("integration vector").

"Cloning vectors" typically contain one or a small number of restriction endonuclease recognition sites at which foreign DNA sequences can be inserted in a determinable fashion without loss of essential biological function of the vector, as well as a marker gene that is suitable for use in the identification and selection of cells transformed with the cloning vector.

The term "expression vector" refers to a DNA molecule, linear or circular, that comprises a segment encoding a polypeptide of interest under the control of (*i.e.* operably linked to) additional nucleic acid segments that provide for its transcription. Such additional segments may include promoter and terminator sequences, and may optionally include one or more origins of replication, one or more selectable markers, an enhancer, a polyadenylation signal, and the like. Expression vectors are generally derived from plasmid or viral DNA, or may contain elements of both. In particular an expression vector comprises a nucleotide sequence that comprises in the 5' to 3' direction and operably linked: (a) a transcription and translation initiation region that are recognized by the host organism, (b) a coding sequence for a polypeptide of interest, and (c) a transcription and translation termination region that are recognized by the host organism. "Plasmid" refers to autonomously replicating extrachromosomal DNA which is not integrated into a microorganism's genome and is usually circular in nature.

An "integration vector" refers to a DNA molecule, linear or circular, that can be incorporated into a microorganism's genome and provides for stable inheritance of a gene encoding a polypeptide of interest. The integration vector generally comprises one or more segments comprising a gene sequence encoding a polypeptide of interest under the control of (*i*.*e*., operably linked to) additional nucleic acid segments that provide for its transcription. Such additional segments may include promoter and terminator sequences, and one or more segments that drive the incorporation of the gene of interest into the genome of the target cell, usually by the process of homologous recombination. Typically, the integration vector will be one which can be transferred into the target cell, but which has a replicon which is non-functional in that organism. Integration of the segment comprising the gene of interest may be selected if an appropriate marker is included within that segment.

One or more nucleic acid sequences encoding appropriate signal peptides that are not naturally associated with a polypeptide to be expressed in a host cell of the invention can be incorporated into (expression) vectors. For example, a DNA sequence for a signal peptide leader can be fused in-frame to a nucleic acid sequence so that the polypeptide is initially translated as a fusion protein comprising the signal peptide. Depending on the nature of the signal peptide, the expressed polypeptide will be targeted differently. A secretory signal peptide that is functional in the intended host cells, for instance, enhances extracellular secretion of the expressed polypeptide. Other signal peptides direct the expressed polypeptide to certain organelles, like the chloroplasts, mitochondria and peroxisomes. The signal peptide can be cleaved from the polypeptide upon transportation to the intended organelle or from the cell. It is possible to provide a fusion of an additional peptide sequence at the amino or carboxyl terminal end of the polypeptide.

The term "functional homologue" of an amino acid sequence, or in short "homologue", as used herein, refers to a polypeptide comprising said specific sequence with the proviso that one or more amino acids are substituted, deleted, added, and/or inserted, and which polypeptide has (qualitatively) the same enzymatic functionality for substrate conversion

The term "functional homologue" of a nucleic acid sequence is used for nucleic acid sequences encoding the same polypeptide as said nucleic acid sequence.

Sequence identity, homology or similarity is defined herein as a relationship between two or more polypeptide sequences or two or more nucleic acid sequences, as determined by comparing those sequences. Usually, sequence identities or similarities are compared over the whole length of the sequences, but may however also be compared only for a part of the sequences aligning with each other. In the art, "identity" or "similarity" also means the degree of sequence relatedness between polypeptide sequences or nucleic acid sequences, as the case may be, as determined by the match between such sequences. Sequence identity as used herein is the value as determined by the EMBOSS Pairwise Alignment Algoritm "Needle". In particular, the NEEDLE program from the EMBOSS package can be used (version 2.8.0 or higher, EMBOSS: The European Molecular Biology Open Software Suite - Rice, P., et al. Trends in Genetics (2000) 16: 276-277; http://emboss.bioinformatics.nl/) using the NOBRIEF option ('Brief identity and similarity' to NO) which calculates the "longest-identity".

The identity, homology or similarity between the two aligned sequences is calculated as follows: Number of corresponding positions in the alignment showing an identical amino acid in both sequences divided by the total length of the alignment after subtraction of the total number of gaps in the alignment. For alignment of amino acid sequences the default parameters are: Matrix = Blosum62; Open Gap Penalty = 10.0; Gap Extension Penalty = 0.5. For alignment of nucleic acid sequences the default parameters are: Matrix = DNAfull; Open Gap Penalty = 10.0; Gap Extension Penalty = 0.5. Discrepancies between a monoterpene synthase according to a specfic sequence or a nucleic acid according to a specific sequence on hand and a functional homologue of said enzyme or nucleic acid may in particular be the result of modifications performed, *e.g.* to improve a property of the enzyme or nucleic acid (*e.g.* improved expression) by a biological technique known to the skilled person in the art, such as *e*.*g*. molecular evolution or rational design or by using a mutagenesis technique known in the art (random mutagenesis, site-directed mutagenesis, directed evolution, gene recombination, *etc*.). The enzyme's or the nucleic acid's sequence may be altered, as a result of one or more natural occurring variations. Examples of such natural modifications/variations are differences in glycosylation (more broadly defined as "post-translational modifications"), differences due to alternative splicing, and single-nucleic acid polymorphisms (SNPs). The nucleic acid may be modified such that it encodes a polypeptide that differs by at least one amino acid, so that it encodes a polypeptide comprising one or more amino acid substitutions, deletions and/or insertions, which polypeptide still has the desired (original) monoterpene synthase activity. Further, use may be made of artificial gene-synthesis (synthetic DNA). Further, use may be made of codon optimisation or codon pair optimisation, *e*.*g*. based on a method as described in WO 2008/000632 or as offered by commercial DNA synthesizing companies like DNA2.0, Geneart, and GenScript.

A host cell according to the invention may be produced based on standard genetic and molecular biology techniques that are generally known in the art, *e.g.* as described in Sambrook, J., and Russell, D.W. "Molecular Cloning: A Laboratory Manual" 3d ed., Cold Spring Harbor Laboratory Press, Cold Spring Harbor, NY, (2001); and F.M. Ausubel et al, eds., "Current protocols in molecular biology", John Wiley and Sons, Inc., New York (1987), and later supplements thereto.

In general, the host cell is an organism comprising genes for expressing the enzymes for catalysing the reaction steps of the mevalonate pathway enabling the production of the C5 prenyl diphosphates isopentenyl diphosphate (IPP). In particular, the host cell comprises genes for expressing the following enzymes of the mevalonate pathway:
(i) an enzyme having catalytic activity in the reaction of acetoacyl-CoA with acetyl-CoA to form HMG-CoA ;
(ii) an enzyme having catalytic activity in the conversion of HMG-CoA to mevalonate;
(iii) an enzyme having catalytic activity in the phosphorylisation of mevalonate to mevalonate 5-phosphate;
(iv) an enzyme having catalytic activity in the conversion of mevalonate 5-phosphate to mevalonate 5-pyrophosphate;
(v) an enzyme having catalytic activity in the conversion of mevalonate 5-pyrophosphate to IPP; and
(vi) an enzyme having catalytic activity in the reversible conversion of IPP to DMAPP.

The genes encoding enzymes (i), (ii), (iii), (iv) and (v) are usually heterologous. Preferably, one or more homologous genes encoding enzyme (vi), having catalytic activity in the reversible conversion of IPP to DMAPP, is present. In addition, one or more heterologous genes encoding an enzyme (vi) may advantageously be present.

The *Rhodobacter* host cell typically comprises one or more homologous genes encoding a homologous enzyme having catalytic activity in the reaction of conversion of two molecules of acetyl-CoA to acetoacyl-CoA (hereafter 'thiolase'). Preferably, the host cell only comprises the thiolase encoded by one or more homologous thiolase genes in case of a *Rhodobacter* for producing a monoterpene.

The host cell comprises a prenyl transferase having catalytic activity for the condensation of IPP and DMAPP into geranyl diphosphate (GPP). Depending on the specific prenyl transferase this enzyme also catalyses the further condensation of IPP and GPP into farnesyl diphosphate (FPP). FPP is the substrate for sesquiterpene synthases and GPP is the substrate for monoterpene synthases. GPP and FPP synthesis can be enhanced by the expression of heterologous GPP or FPP synthases, respectively. Bacterial enzymes usually catalyse both condensations and thus are useful for the production of sesquiterpenes. Specific GPP synthases do not produce FPP and are thus particularly useful for the production of monoterpenes. Many GPP synthases have been described form plants and heterologous expression of such enzymes are useful for the production of monoterpenes in bacterial of fungal cells.

The *Rhodobacter* host cell is preferably selected from the group of *Rhodobacter capsulatus* and *Rhodobacter sphaeroides.*

The term "monoterpende synthase" is used herein for polypeptides having catalytic activity in the formation of a monoterpene from geranyl pyrophosphate, and for other moieties comprising such a polypeptide. Examples of such other moieties include complexes of said polypeptide with one or more other polypeptides, other complexes of said polypeptides (*e*.*g*. metalloprotein complexes), macromolecular compounds comprising said polypeptide and another organic moiety, said polypeptide bound to a support material, *etc.* The monoterpene synthase can be provided in its natural environment, *i.e.* within the cell in which it has been produced, or in the medium into which it has been excreted by the cell producing it. It can also be provided separate from the source that has produced the polypeptide and can be manipulated by attachment to a carrier, labeled with a labeling moiety, and the like.

Suitable monoterpene synthases can be based on those known in the art. For instance use may be made of the monoterpene synthases mentioned in or referred to in US 7,659,097, in particular column 11, line 25 - column 15, line 5.

Preferably, the enzyme having monoterpene synthase activity is selected from the group of enzymes having beta-pinene synthase activity, alpha-pinene synthase activity, myrcene synthase activity, limonene synthase activity (in particular, L-(-)limonene synthase activity), sabinene synthase activity, and geraniol synthase activity.

In a particularly preferred embodiment, the enzyme having monoterpene synthase activity has beta-pinene synthase activity and is a beta-pinene synthase comprising a sequence having at least 40 %, at least 50 %, at least 60 %, at least 70 %, at least 80 %, at least 85 %, at least 90 %, at least 95 % at least 96 %, at least 97 %, at least 98 5 or at least 99 % sequence identity with SEQ ID NO: 2.

Preferably, said beta-pinene synthase comprises a NALI motive. This motive is preferably present in the region corresponding to position 271-315 of SEQ ID NO: 2, in particular in the region corresponding to position 281-305 of SEQ ID NO: 2, more in particular in the region corresponding to position 291-295 of SEQ ID NO: 2.

Preferably, the beta-pinene synthase comprises a IGATV motive. This motive is preferably present in the region corresponding to position 380-424 of SEQ ID NO: 2, in particular in the region corresponding to position 390-414 of SEQ ID NO: 2, more in particular in the region corresponding to position 400-404 of SEQ ID NO: 2.

The NALI and/or the IGATV motive are preferably present, for a high product specificity.

Preferably, the beta-pinene synthase comprises a RRXₛW and/or a DDXXD motive, wherein each X can be selected from the group of proteinogenic amino acids (the amino acids encoded by codons of DNA/RNA) and s is an integer in the range of 4 to 12, in particular 7, 8 or 9, preferably 8. The proteinogenic amino acids may in particular be selected from S, A. D, Y, G, P, T and I (using the standard 1-letter code). In a specific embodiment s=8 and the amino-acid sequence of Xₛ = Xs = SADYGPTI.

The presence of a DDXXD is in particular preferred for metal binding (magnesium ion binding) to form a well functioning beta-pinene synthase.

The RRXₛW demarks the start of the mature protein. In the citrus plant enzyme from which Sequence ID NO 2 is derived (gene bank accession number AF514288) This sequence is preceded by a choroplast targeting signal (MALNLLSSIPAACNFTRLSLPLSSKVNGFVPPITRVQYHVAASTTPIKPVDQTII).

The monoterpene synthase, in particular the beta-pinene synthase, expressed in a host cell according to the invention is preferably free of a chloroplast targeting signal upstream of the RRXₛW motive.

A nucleic acid sequence encoding the beta-pinene synthase of Sequence ID NO: 2 is shown in Sequence ID NO: 1.

Another suitable pinene synthase is a beta-pinene synthase comprising a sequence having at least 30 %, in particular at least 40 %, at least 50 %, at least 60 %, at least 70 %, at least 80 %, at least 85 %, at least 90 %, at least 95 % at least 96 %, at least 97 %, at least 98 % or at least 99 % sequence identity with SEQ ID NO: 4. A nucleic acid sequence encoding the beta-pinene synthase of Sequence ID NO: 4 is shown in Sequence ID NO: 3.

Another suitable pinene synthase is a pinene synthase comprising a sequence having at least 30 %, in particular at least 40 %, at least 50 %, at least 60 %, at least 70 %, at least 80 %, at least 85 %, at least 90 %, at least 95 % at least 96 %, at least 97 %, at least 98 % or at least 99 % sequence identity with SEQ ID NO: 6. A nucleic acid sequence encoding the pinene synthase of Sequence ID NO: 6 is shown in Sequence ID NO: 5. Such pinene synthase may also in particular be used for alpha-pinene synthesis.

Specific examples of geraniol synthases are shown in Sequence ID NO: 12, 14 and 16. The host cell may in particular comprise such genaniol synthase or a geraniol synthase having at least 30 %, in particular at least 40 %, at least 50 %, at least 60 %, at least 70 %, at least 80 %, at least 85 %, at least 90 %, at least 95 % at least 96 %, at least 97 %, at least 98 % or at least 99 % sequence identity with Sequence ID NO: 12, 14 or 16. Examples of encoding nucleic acids are shown in Sequence ID NO: 11, 13 and 15, respectively.

Specific examples of myrcene synthases are shown in Sequence ID NO: 18, and 20. The host cell may in particular comprise such myrcene synthase or a myrcene synthase having at least 30 %, in particular at least 40 %, at least 50 %, at least 60 %, at least 70 %, at least 80 %, at least 85 %, at least 90 %, at least 95 % at least 96 %, at least 97 %, at least 98 % or at least 99 % sequence identity with Sequence ID NO: 18 or 20. Examples of encoding nucleic acids are shown in Sequence ID NO: 17 and 19, respectively.

The term "sesquiterpene synthase" is used herein for polypeptides having catalytic activity in the formation of sesquiterpene from farnesyl diphosphate, and for other moieties comprising such a polypeptide. Examples of such other moieties include complexes of said polypeptide with one or more other polypeptides, other complexes of said polypeptides (*e*.*g*. metalloprotein complexes), macromolecular compounds comprising said polypeptide and another organic moiety, said polypeptide bound to a support material, *etc.* The sesquiterpene synthase can be provided in its natural environment, *i.e.* within a cell in which it has been produced, or in the medium into which it has been excreted by the cell producing it. It can also be provided separate from the source that has produced the polypeptide and can be manipulated by attachment to a carrier, labeled with a labeling moiety, and the like.

Suitable sesquiterpene synthases can be based on those known in the art. For instance use may be made of the terpene synthases mentioned in or referred to in US 7,659,097.

In particular, the sesquiterpene synthase may be selected from the group of valencene synthases, santalene synthases and patchoulol synthases.

Preferably, the sesquiterpene synthase is a valencene synthase. For instance the valencene synthase gene in the host cell may originate from *Citrus x paradisi.* Preferably, the valencene synthase comprises a sequence having at least 30 %, in particular at least 40 %, at least 50 %, at least 60 %, at least 70 %, at least 80 %, at least 85 %, at least 90 %, at least 95 % or at least 98 % sequence identity with SEQ ID NO: 8. The valencene synthase of SEQ ID NO: 8 or a functional homologue thereof in particular has an improved specificity and/or productivity compared to valencene synthase from *Citrus x paradisi*, with respect to catalysing the conversion of FPP into valencene.

Preferably, the valencene synthase comprises a sequence according to SEQ ID NO: 9, preferably according to SEQ ID NO: 10, with the proviso that it is different from the valencene synthase of SEQ ID NO: 8. such valencene synthase may in particular improved productivity, preferably improved specific productivity, towards the conversion of farnesyl diphosphate into valencene, compared to a valencene synthase having the amino acid sequence of SEQ ID NO: 8.

Preferably, the valencene synthase comprises an amino acid sequence as shown in SEQ ID NO: 9 or a functional homologue thereof. Compared to the valencene synthase of SEQ ID NO: 8, the valencene synthase with a sequence according to SEQ ID NO: 9 is in particular preferred for its improved productivity, in particular its improved specific productivity.

Herein, the positions marked with an 'X' can in principle contain any amino acid residue, with the proviso that preferably at least one amino acid residue is different from the corresponding amino acid residue in SEQ ID NO: 8. Preferably, the valencene synthase comprises an amino acid sequence as shown in SEQ ID NO: 10 or a functional homologue thereof. Herein, the particularly preferred amino acid residues are given between parenthesis for positions that have been marked with an 'X' in SEQ ID NO: 9. Preferably at least one of these positions has an amino acid residue different from the corresponding amino acid residue in SEQ ID NO: 8.

Specifically, the valencene synthase with improved productivity, in particular a functional homologue of the valencene synthase of SEQ ID NO: 8, comprises a modification at one or more of the positions aligning with: 87, 93, 128, 171, 178, 187, 226, 302, 312, 319, 323, 398, 436, 448, 449, 450, 463, 488, 492, 502, 507, 530 or 559 of SEQ ID NO: 8.

Preferably, the valencene synthase has one or more modifications, in particular one or more substitutions, compared to the valence synthase represented by SEQ ID NO: 8, at an amino acid position corresponding to a position selected from the group of 16, 128, 171, 187, 225, 244, 300, 302, 307, 319, 323, 327, 331, 334, 398, 405, 409, 410, 412, 436, 438, 439, 444, 448, 449, 450, 463, 488, 490, 492, 502, 503, 507, 527, 556, 559, 560, 566, 568, 569, and 570 of SEQ ID NO: 8. More preferably, the valencene synthase comprises one or more modifications at a position corresponding to (aligning with) one or more positions selected from the group of 16, 225,244, 300, 302, 307, 323, 327, 331, 334, 405, 409, 410, 412, 436, 438, 439, 444, 448, 449, 450, 463, 488, 490, 492, 502, 503, 507, 527, 556, 559, 560, 566, 568, 569, and 570 of SEQ ID NO: 8.

Particularly preferred, the valencene synthase has one or more modifications selected from the group of 16A, 16T, 16S, 128L, 171R, 187K, 225S, 244S. 244T, 300Y, 302D, 307T, 307A, 319Q, 323A, 327L, 331G, 334L, 398I, 398M, 398T, 405T, 405V, 409F, 410F, 410V, 410L, 412G, 436L, 436K, 436T, 436W, 438T, 439G, 439A, 444I, 444V, 448S, 449F, 449I, 449Y, 450L, 450M, 450V, 463E, 463S, 463G, 463W, 488Y, 488H, 488S, 490N, 490A, 490T, 490F, 492A, 492K, 502Q, 503S, 507E, 507Q, 527T, 527S, 527A, 556T, 559H, 559L, 559V, 560L, 566S, 566A, 566G, 568S, 569I, 569V,570T, 570G, 570A and 570P.

In particular, preferred is a valencene synthase having one or more modifications selected from the group of 16A, 16T, 16S, 244S, 244T, 300Y, 307T, 307A, 323A, 327L, 331G, 334L, 405T, 405V, 409F, 410F, 410V, 410L, 412G, 436L, 436K, 436T, 438T, 439G, 439A, 444I, 448S, 449F, 450M, 450L, 450V, 463E, 463S, 463G, 463W, 488Y, 488S, 488H, 490N, 490A, 490T, 490F, 492A, 492K, 502Q, 503S, 507E, 507Q, 527T, 527S, 527A, 556T, 559H, 559L, 559V, 560L, 566S, 566A, 566G, 568S, 569I, 569V, 570T, 570G and 570P.

For a particularly high productivity a valencene synthase having at least one modification selected from the group of 16A, 244S, 300Y, 307T, 307A, 323A, 327L, 331G, 334L, 405T, 409F, 410F, 410V, 410L, 412G, 436L, 436K, 436T, 438T, 439G, 439A, 449F, 450L, 450V, 488Y, 488H, 488S, 490N, 490A, 490T, 492A, 492K, 502Q, 503S, 507E, 507Q, 527T, 556T, 559H, 559L, 560L, 566S, 566A, 568S, 569I, 569V, 570T and 570G is preferred.

Preferred examples of valencene synthases comprising at least two modifications compared to SEQ ID NO: 8 are those wherein at least two modifications are selected from 128L, 187K, 302D, 398I, 398M, 398T, 436L, 436K, 436W, 449F, 449I, 449Y, 450L, 450F, 450V, 463E, 463S, 463G, 463W, 488S, 488Y and 488H.

Although good results can be obtained with valencene synthases having only one or two mutations (substitutions) compared to SEQ ID NO: 8, the valence synthase may comprise more modifications, in particular three or more, four or more, five or more, six or more or seven or more modifications. In principle there is no limit to the number of modifications, provided that the enzyme retains sufficient catalytic properties as a valencene synthase.

The monoterpene synthase or the sesquiterpene synthase may consist of a polypeptide referred to herein above. However, it is also possible that the monoterpene synthase or the sesquiterpene synthase comprises at least one segment having such polypeptide, e.g. with a sequence identity as shown in the list of sequences, or sequence having a high sequence identity therewith, such as a sequence identity of more than 30 %, more than 50 %, more than 70 % or more than 80 %, and at least one further peptide segment, such as a tag peptide.

Thus, in a specific embodiment, the host cell is capable of expressing a polypeptide with monoterpene synthase activity, the polypeptide comprising a first polypeptide segment and a second polypeptide segment, the first segment comprising a tag-peptide and the second segment comprising a polypeptide providing the monoterpene synthase activity.

The tag-peptide is preferably selected from the group of nitrogen utilization proteins (NusA), thioredoxins (Trx) and maltose-binding proteins (MBP). Moreover small peptides with large net negative charge, as have been described by Zhang, Y-B, et al., Protein Expression and Purification (2004) 36: 207-216, can be used as tag-peptide. Particularly suitable is maltose binding protein from *Escherichia coli.* The tag may in particular improve productivity of the enzyme, by increasing the expression of the monoterpene synthase in active form. Preferably, the monoterpene synthase having a tag-peptide segment has an increased specific productivity, increased stability or an increased product specificity, in particular if the tag-peptide is selected from the group of nitrogen utilization proteins (NusA), thioredoxins (Trx) and maltose-binding proteins (MBP).

For improved solubility of the tagged monoterpene synthase (compared to said synthase without the tag), the first segment of the tagged monoterpene synthase s preferably bound at its C-terminus to the N-terminus of the second segment. Alternatively, the first segment of the tagged monoterpene synthase is bound at its N-terminus to the C-terminus of the second segment.

The *Rhodobacter* host cell according to the invention can be used industrially in the production of a monoterpene, preferably a monoterpene selected from the group of beta-pinene, myrcene, alpha-pinene, limonene, sabinene, and geraniol.

In principle, the production of the monoterpene can be carried out in a manner based on methodology known *per se*, *e.g.* as described in the prior art mentioned herein above. The host cell may be used in a fermentative production of the montoterpene, or it may be used to produce a monoterpene synthase, which can thereafter then be used for synthesis of the desired terpenoid.

Advantageously, the monoterpene is produced in a fermentative process, *i.e.* in a method comprising cultivating the *Rhodobacter* host cells in a culture medium under conditions wherein the monoterpene synthase is expressed. The actual reaction catalysed by the monoterpene synthase typically takes place intracellularly.

It should be noted that the term "fermentative" is used herein in a broad sense for processes wherein use is made of a culture of an organism to synthesise a compound from a suitable feedstock (*e.g.* a carbohydrate, an amino acid source, a fatty acid source). Thus, fermentative processes as meant herein are not limited to anaerobic conditions, and extended to processes under aerobic conditions. Suitable feedstocks are generally known for *Rhodobacter* host cells. Suitable conditions may be based on known methodology for *Rhodobacter* host cells, *e.g.* described in WO 2011/074954 (in particular page 68 (examples, general part, shake-flask procedure), the information disclosed herein, common general knowledge and optionally some routine experimentation.

In principle, the pH of the reaction medium (culture medium) used in a method according to the invention may be chosen within wide limits, as long as the host cell is active and displays a wanted specificity under the pH conditions. In case the method includes the use of cells, for expressing the valencene synthase, the pH is selected such that the cells are capable of performing their intended function or functions. The pH may in particular be chosen within the range of four pH units below neutral pH and two pH units above neutral pH, *i.e.* between pH 3 and pH 9 in case of an essentially aqueous system at 25°C. Good results have e.g. been achieved in an aqueous reaction medium having a pH in the range of 6.8 to 7.5.

A system is considered aqueous if water is the only solvent or the predominant solvent (> 50 wt. %, in particular > 90 wt. %, based on total liquids), wherein e.g. a minor amount of alcohol or another solvent (< 50 wt. %, in particular < 10 wt. %, based on total liquids) may be dissolved (*e.g.* as a carbon source, in case of a full fermentative approach) in such a concentration that micro-organisms which are present remain active.

The reaction conditions can be aerobic, oxygen-limited or anaerobic.

Anaerobic conditions are herein defined as conditions without any oxygen or in which substantially no oxygen is consumed by the cultured cells, in particular a micro-organism, and usually corresponds to an oxygen consumption of less than 5 mmol/l.h, preferably to an oxygen consumption of less than 2.5 mmol/l.h, or more preferably less than 1 mmol/l.h. Aerobic conditions are conditions in which a sufficient level of oxygen for unrestricted growth is dissolved in the medium, able to support a rate of oxygen consumption of at least 10 mmol/l.h, more preferably more than 20 mmol/l.h, even more preferably more than 50 mmol/l.h, and most preferably more than 100 mmol/l.h.

Oxygen-limited conditions are defined as conditions in which the oxygen consumption is limited by the oxygen transfer from the gas to the liquid. The lower limit for oxygen-limited conditions is determined by the upper limit for anaerobic conditions, *i.e.* usually at least 1 mmol/l.h, and in particular at least 2.5 mmol/l.h, or at least 5 mmol/l.h. The upper limit for oxygen-limited conditions is determined by the lower limit for aerobic conditions, *i.e.* less than 100 mmol/l.h, less than 50 mmol/l.h, less than 20 mmol/l.h, or less than to 10 mmol/l.h.

Whether conditions are aerobic, anaerobic or oxygen-limited is dependent on the conditions under which the method is carried out, in particular by the amount and composition of ingoing gas flow, the actual mixing/mass transfer properties of the equipment used, the type of *Rhodobacter* used and the microorganism density.

In principle, the temperature used is not critical, as long as the cells, show substantial activity. Generally, the temperature may be at least 0°C, in particular at least 15°C, more in particular at least 20°C. A desired maximum temperature depends upon the enzymes and the cells. The temperature is generally 70°C or less, preferably 50°C or less, more preferably 40°C or less, in particular 35°C or less.

In particular if the catalytic reaction whereby monoterpene is formed, is carried out outside a host cell, a reaction medium comprising an organic solvent may be used in a high concentration (*e*.*g*. more than 50 %, or more than 90 wt. %, based on total liquids), in case the monoterpene that is used retains sufficient activity and specificity in such a medium.

If desired, the monoterpene produced in a method according to the invention, or a further compound into which the monoterpene has been converted after its preparation, is recovered from the reaction medium, wherein it has been made. A suitable method usually is liquid-liquid extraction with an extracting liquid that is non-miscible with the reaction medium.

In an advantageous embodiment, the monoterpene (or a further product) is produced in a reactor comprising a first liquid phase (the reaction phase), said first liquid phase containing cells according to the invention in which cells the monoterpene (or a further product) is produced, and a second liquid phase (organic phase that remains essentially phase-separated with the first phase when contacted), said second liquid phase being the extracting phase, for which the formed product has a higher affinity. This method is advantageous in that it allows *in situ* product recovery. Also, it contributes to preventing or at least reducing potential toxic effects of the monoterpene (or a further product) to the cells, because due to the presence of the second phase, the monoterpene (or a further product) concentration in the reaction phase may be kept relatively low throughout the process. Finally, there are strong indications that the extracting phase contributes to extracting the monoterpene (or further product) out of the reaction phase.

In a preferred method of the invention the extracting phase forms a layer on top of the reaction phase or is mixed with the reaction phase to form a dispersion of the reaction phase in the extracting phase or a dispersion of the extracting phase in the reaction phase. Thus, the extracting phase not only extracts product from the reaction phase, but also helps to reduce or completely avoid losses of the formed product from the reactor through the off-gas, that may occur if the monoterpene is produced in the (aqueous) reaction phase or excreted into the (aqueous) reaction phase. Generally, monoterpenes are poorly soluble in water and therefore easily volatilize from water. It is contemplated that a monoterpene dissolved in the organic phase is at least substantially prevented from volatilization.

Suitable liquids for use as extracting phase combine a lower density than the reaction phase with a good biocompatibility (no interference with the viability of living cells), low volatility, and near absolute immiscibility with the aqueous reaction phase. Examples of suitable liquids for this application are liquid alkanes like decane, dodecane, isododecane, tetradecane, and hexadecane or long-chain aliphatic alcohols like oleyl alcohol, and palmitoleyl alcohol, or esters of long-chain fatty acids like isopropyl myristate, and ethyl oleate (see *e.g.* Asadollahi et al. (Biotechnol. Bioeng. (2008) 99: 666-677), Newman et al. (Biotechnol. Bioeng. (2006) 95: 684-691) and WO 2009/042070).

The monoterpene produced in accordance with the invention may be used as such, *e.g.* for use as a flavour or fragrance, or as an insect repellent, or may be used as a starting material for another compound, in particular another flavour or fragrance..
SEQUENCE ID NO 1:
   *beta-pinene synthase*
   from Citrus limon, >gi|21435705|gb|AAM53945.1|AF514288_1 (-)-beta-pinene synthase
   nucleotide sequence 
AGGCGATCTGCTGATTACGGCCAACCATTTGGAGTTTTGATTATATTCAATCACTTGACAGTAAA
TATAAAGGAGAATCGTATGCCAGACAACTGGAAAAGCTGAAGGAACAAGTAAGCGCGATGCTACAG
CAGGATAATAAAGTGGTGGATTTGGATACTTTACATCAACTTGAGCTCATCGATAATCTGCACAGA
CTTGGAGTATCTTATCACTTTGAGGATGAAATAAAAAGAACTTTGGATAGGATACACAACAAGAAT
ACAAATAAAAGTTTATATGCCACAGCACTCAAATTTAGAATCCTAAGGCAATATGGTTACAATACA
CCTGTAAAAGAAACTTTTTCACGTTTCATGGATGAGAAAGGGAGCTTTAAGTCATCAAGCCACAGT
GACGACTGCAAAGGAATGTTAGCTCTGTATGAAGCCGCATACCTCCTGGTAGAAGAAGAAAGCAGT
ATCTTTCGTGATGCTAAAAGTTTCACCACCGCATATCTCAAAGAATGGGTAATCGAGCATGATAAT
AATAAACATGATGATGAACATCTTTGTACATTAGTGAATCATGCTTTGGAACTTCCACTACATTGG
AGGATGCCAAGATTGGAGGCAAGGTGGTTCATCGATGTGTACGAAAATGGACCACACATGAACCCT
ATCTTGCTCGAGCTTGCTAAAGTTGACTTTAATATTGTGCAAGCAGTACACCAAGAGAATCTCAAA
TATGCATCAAGGTGGTGGAAGAAAACAGGACTTGGGGAGAATTTGAATTTTGTAAGAGACAGAATA
GTGGAGAATTTCATGTGGACGGTGGGGGAGAAATTCGAACCTCAGTTTGGATATTTTAGACGGATG
TCTACAATGGTCAATGCCTTAATAACAGCAGTCGATGATGTTTATGATGTCTACGGACTTTGGAG
GAACTTGAGATATTCACTGATGCAGTTGAGAGATGGGACGCTACTGCAGTAGAGCAACTTCCACAC
TATATGAAGTTGTGCTTTCATGCTCTCCGTAATTCCATAAATGAAATGACTTTTGATGCTCTTAGG
GATCAAGGAGTTGACATTGTCATTTCTTATCTTACGAAAGCGTGGGCAGATATATGTAAAGCATAT
TTAGTAGAGGCAAAGTGGTACAACAGCGGCTACATACCGCCTCTCCAAGAATACATGGAAAATGCT
TGGATTTCAATAGGAGCAACTGTAATTCTAGTCCATGCAAACACTTTTACAGCAAATCCAATAACA
AAGGAGGGCTTGGAATTCGTGAAAGATTATCCCAATATAATTCGTTGGTCATCGATGATTCTACGG
TTTGCAGACGATTTGGGAACATCATCGGATGAGCTGAAGAGGGGAGATGTTCATAAATCAATTCAA
TGTTACATGCATGAAGCTGGAGTTTCAGAGGGAGAGGCTCGTGAACATATAAATGATTTGATTGCT
CAGACATGGATGAAGATGAACCGTGATCGATTTGGAAACCCACATTTCGTTTCCGACGTTTTTGTT
GGGATTGCAATGAATTTGGCGAGGATGTCTCAATGCATGTACCAATTTGGAGATGGTCACGGATGC
GGTGCTCAAGAAATTACTAAAGCTCGTGTTTTGTCCTTATTTTTTGATCCCATTGCTTAA
SEQUENCE ID NO 2:
   *beta-pinene synthase*
   from Citrus limon, >gi|21435705|gb|AAM53945.1|AF514288_1 (-)-beta-pinene synthase
   amino acid sequence 
RRSADYGPTIWSFDYIQSLDSKYKGESYARQLEKLKEQVSAMLQQDNKVV
DLDTLHQLELIDNLHRLGVSYHFEDEIKRTLDRIHNKNTNKSLYATALKF
RILRQYGYNTPVKETFSRFMDEKGSFKSSSHSDDCKGMLALYEAAYLLVE
EESSIFRDAKSFTTAYLKEWVIEHDNNKHDDEHLCTLVNHALELPLHWRM
PRLEARWFIDVYENGPHMNPILLELAKVDFNIVQAVHQENLKYASRWWKK
TGLGENLNFVRDRIVENFMWTVGEKFEPQFGYFRRMSTMVNALITAVDDV
YDVYGTLEELEIFTDAVERWDATAVEQLPHYMKLCFHALRNSINEMTFDA
LRDQGVDIVISYLTKAWADICKAYLVEAKWYNSGYIPPLQEYMENAWISI
GATVILVHANTFTANPITKEGLEFVKDYPNIIRWSSMILRFADDLGTSSD
ELKRGDVHKSIQCYMHEAGVSEGEAREHINDLIAQTWMKMNRDRFGNPHF
VSDVFVGIAMNLARMSQCMYQFGDGHGCGAQEITKARVLSLFFDPIA
SEQUENCE ID NO: 3
   beta-pinene synthase
   from Artemisia annua >gi|14279758|gb|AAK58723.1|AF276072_1 (-)-beta-pinene synthase [Artemisia annua]
   nucleotide sequence 
AGAAGATCAGCTAATTATGCCCCTTCATTATGGTCCTATGATTTTGTCCAGTCGCTTTCTAGCAAA
TACAAAGGAGATAACTATATGGCAAGATCACGAGCTCTAAAAGGAGTAGTGAGGACCATGATTTTA
GAAGCGAATGGAATTGAAAATCCATTGAGTTTACTTAATTTGGTCGATGATTTGCAAAGACTTGGA
ATATCATATCATTTTTTGGATGAAATAAGCAATGTTTTGGAGAAAATATACTTAAATTTCTACAAA
AGTCCTGAAAAGTGGACTAATATGGATTTAAATCTTAGATCCCTTGGTTTTAGACTCTTGAGACAA
CATGGATATCATATTCCTCAAGAGATATTCAAGGACTTTATAGACGTGAATGGAAATTTCAAGGGA
GATATCATCAGCATGCTAAATTTGTATGAAGCTTCTTATCATTCAGTAGAGGAGGAAAGTATATTG
GATGATGCTAGAGAGTTCACAACAAAATATTTGAAAGAAACTTTAGAGAATATTGAAGATCAAAAT
ATAGCGTTGTTCATAAGTCATGCATTGGTTTTCCACTTCATTGGATGGTTCCACGGGTGGAAACA
AGTTGGTTTATTGAAGTTTATCCGAAAAAGTTGGCATGAATCCCACGGTGCTTGAGTTTGCGAAA
CTGGACTTCAACATACTGCAGGCAGTTCACCAAGAAGATATGAAAAAGCATCAAGATGGTGGAAA
GAAACATGCTGGGAGAAGTTTGGCTTTGCTCGTGATCGTTTGGTGGAGAACTTCATGTGGACTGTT
 
GCCGAAAATTACTTGCCTCATTTTCAAACAGGAAGGGGAGTTCTCACAAAGGTTAACGCCATGATA
ACCACTATCGACGATGTTTATGATGTGTATGGTACTTTGCCTGAACTCGAACTATTTACCAACATT
GTAAACAGTTGGGATATCAATGCGATTGATGAACTTCCGGATTATTTGAAAATATGCTTCCTTGCG
TGCTACAATGCTACCAATGAATTATCATATAACACATTGACAAACAAAGGATTCTTCGTACATCCT
TACCTTAAAAAGGCGTGGCAGGATTTATGCAACTCTTACATAATTGAAGCTAAATGGTTCAATGAT
GGATACACACCAACCTTCAACGAGTTCATTGAAAATGCATACATGTCAATAGGAATTGCTCCGATC
ATCAGGCATGCCTATTTGTTAACATTAACTAGTGTTACCGAAGAAGCATTGCAACACATAGAAAGA
GCTGAAAGTATGATTCGCAATGCATGCCTAATTGTGCGACTCACTAATGATATGGGCACATCATCT
GATGAGCTTGAAAGAGGTGATATTCCAAAATCAATCCAGTGCTATATGCACGAAAGTGGTGCTACT
GAAATGGAAGCACGAGCGTATATAAAACAGTTCATCGTCGAGACATGGAAGAAACTGAACAAAGAA
CGGCAAGAAATTGGTTCTGAATTTCCGCAAGAGTTCGTTGATTGTGTTATAAACCTTCCTAGAATG
GGTCATTTCATGTATACCGATGGAGACAAACATGGTAAACCCGACATGTTCAAGCCGTATGTATTT
TCATTGTTTGTTAATCCAATCTAG
SEQUENCE ID NO 4:
   beta-pinene synthase
   from Artemisia annua >gi|14279758|gb|AAK58723.1|AF276072_1 (-)-beta-pinene synthase [Artemisia annua]
   amino acid sequence 
RRSANYAPSLWSYDFVQSLSSKYKGDNYMARSRALKGVVRTMILEANGIENPLSLLNLVDDLQRLG
ISYHFLDEISNVLEKIYLNFYKSPEKWTNMDLNLRSLGFRLLRQHGYHIPQEIFKDFIDVNGNFKG
DIISMLNLYEASYHSVEEESILDDAREFTTKYLKETLENIEDQNIALFISHALVFPLHWMVPRVET
SWFIEVYPKKVGMNPTVLEFAKLDFNILQAVHQEDMKKASRWWKETCWEKFGFARDRLVENFMWTV
AENYLPHFQTGRGVLTKVNAMITTIDDVYDVYGTLPELELFTNIVNSWDINAIDELPDYLKICFLA
CYNATNELSYNTLTNKGFFVHPYLKKAWQDLCNSYIIEAKWFNDGYTPTFNEFIENAYMSIGIAPI
IRHAYLLTLTSVTEEALQHIERAESMIRNACLIVRLTNDMGTSSDELERGDIPKSIQCYMHESGAT
EMEARAYIKQFIVETWKKLNKERQEIGSEFPQEFVDCVINLPRMGHFMYTDGDKHGKPDMFKPYVF
SLFVNPI
SEQUENCE ID NO 5:
   *pinene synthase*
   from Abies grandis (AG3.18) >gb|U87909.1|AGU87909:6-1892
   nucleotide sequence 
AGACGCATGGGCGATTTCCATTCCAACCTCTGGGACGATGATGTCATACAGTCTTTACCAACGGCT
TATGAGGAAAAATCGTACCTGGAGCGTGCTGAGAAACTGATCGGGGAAGTAAAGAACATGTTCAAT
TCGATGTCATTAGAAGATGGAGAGTTAATGAGTCCGCTCAATGATCTCATTCAACGCCTTTGGATT
GTCGACAGCCTTGAACGTTTGGGGATCCATAGACATTTCAAAGATGAGATAAAATCGGCGCTTGAT
TATGTTTACAGTTATTGGGGCGAAAATGGCATCGGATGCGGGAGGGAGAGTGTTGTTACTGATCTG
AACTCAACTGCGTTGGGGCTTCGAACCCTACGACTACACGGATACCCGGTGTCTTCAGATGTTTTC
AAAGCTTTCAAAGGCCAAAATGGGCAGTTTTCCTGCTCTGAAAATATTCAGACAGATGAAGAGATC
AGAGGCGTTCTGAATTTATTCCGGGCCTCCCTCATTGCCTTTCCAGGGGAGAAAATTATGGATGAG
GCTGAAATCTTCTCTACCAAATATTTAAAAGAAGCCCTGCAAAAGATTCCGGTCTCCAGTCTTTCG
CGAGAGATCGGGGACGTTTTGGAATATGGTTGGCACACATATTTGCCGCGATTGGAAGCAAGGAAT
TACATCCAAGTCTTTGGACAGGACACTGAGAACACGAAGTCATATGTGAAGAGCAAAAAACTTTTA
GAACTCGCAAAATTGGAGTTCAACATCTTTCAATCCTTACAAAAGAGGGAGTTAGAAAGTCTGGTC
AGATGGTGGAAAGAATCGGGTTTTCCTGAGATGACCTTCTGCCGACATCGTCACGTGGAATACTAC
ACTTTGGCTTCCTGCATTGCGTTCGAGCCTCAACATTCTGGATTCAGACTCGGCTTTGCCAAGACG
TGTCATCTTATCACGGTTCTTGACGATATGTACGACACCTTCGGCACAGTAGACGAGCTGGAACTC
TTCACAGCGACAATGAAGAGATGGGATCCGTCCTCGATAGATTGCCTTCCAGAATATATGAAAGGA
GTGTACATAGCGGTTTACGACACCGTAAATGAAATGGCTCGAGAGGCAGAGGAGGCTCAAGGCCGA
GATACGCTCACATATGCTCGGGAAGCTTGGGAGGCTTATATTGATTCGTATATGCAAGAAGCAAGG
TGGATCGCCACTGGTTACCTGCCCTCCTTTGATGAGTACTACGAGAATGGGAAAGTTAGCTGTGGT
CATCGCATATCCGCATTGCAACCCATTCTGACAATGGACATCCCCTTTCCTGATCATATCCTCAAG
GAAGTTGACTTCCCATCAAAGCTTAACGACTTGGCATGTGCCATCCTTCGATTACGAGGTGATACG
CGGTGCTACAAGGCGGACAGGGCTCGTGGAGAAGAAGCTTCCTCTATATCATGTTATATGAAAGAC
AATCCTGGAGTATCAGAGGAAGATGCTCTCGATCATATCAACGCCATGATCAGTGACGTAATCAAA
GGATTAAATTGGGAACTTCTCAAACCAGACATCAATGTTCCCATCTCGGCGAAGAAACATGCTTTT
GACATCGCCAGAGCTTTCCATTACGGCTACAAATACCGAGACGGCTACAGCGTTGCCAACGTTGAA
ACGAAGAGTTTGGTCACGAGAACCCTCCTTGAATCTGTGCCTTTGTAG
SEQUENCE ID NO 6:
   *pinene synthase*
   from Abies grandis
   amino acid sequence 
RRMGDFHSNLWDDDVIQSLPTAYEEKSYLERAEKLIGEVKNMFNSMSLEDGELMSPLNDLIQRLWI
VDSLERLGIHRHFKDEIKSALDYVYSYWGENGIGCGRESVVTDLNSTALGLRTLRLHGYPVSSDVF
KAFKGQNGQFSCSENIQTDEEIRGVLNLFRASLIAFPGEKIMDEAEIFSTKYLKEALQKIPVSSLS
REIGDVLEYGWHTYLPRLEARNYIQVFGQDTENTKSYVKSKKLLELAKLEFNIFQSLQKRELESLV
RWWKESGFPEMTFCRHRHVEYYTLASCIAFEPQHSGFRLGFAKTCHLITVLDDMYDTFGTVDELEL
FTATMKRWDPSSIDCLPEYMKGVYIAVYDTVNEMAREAEEAQGRDTLTYAREAWEAYIDSYMQEAR
WIATGYLPSFDEYYENGKVSCGHRISALQPILTMDIPFPDHILKEVDFPSKLNDLACAILRLRGDT
RCYKADRARGEEASSISCYMKDNPGVSEEDALDHINAMISDVIKGLNWELLKPDINVPISAKKHAF
DIARAFHYGYKYRDGYSVANVETKSLVTRTLLESVPL
SEQUENCE ID NO: 7
   *valC*
   *Chamaecyparis nootkatensis*
   Nucleotide sequence 
ATGGCTGAAATGTTTAATGGAAATTCCAGCAATGATGGAAGTTCTTGCATGCCCGTGAAGGACGCC
CTTCGTCGGACTGGAAATCATCATCCTAACTTGTGGACTGATGATTTCATACAGTCCCTCAATTCT
CCATATTCGGATTCTTCATACCATAAACATAGGGAAATACTAATTGATGAGATTCGTGATATGTTT
TCTAATGGAGAAGGCGATGAGTTCGGTGTACTTGAAAATATTTGGTTTGTTGATGTTGTACAACGT
TTGGGAATAGATCGACATTTTCAAGAGGAAATCAAAACTGCACTTGATTATATCTACAAGTTCTGG
AATCATGATAGTATTTTTGGCGATCTCAACATGGTGGCTCTAGGATTTCGGATACTACGACTGAAT
AGATATGTCGCTTCTTCAGATGTTTTTAAAAAGTTCAAAGGTGAAGAAGGACAATTCTCTGGTTTT
GAATCTAGCGATCAAGATGCAAAATTAGAAATGATGTTAAATTTATATAAAGCTTCAGAATTAGAT
TTTCCTGATGAAGATATCTTAAAAGAAGCAAGAGCGTTTGCTTCTATGTACCTGAAACATGTTATC
AAAGAATATGGTGACATACAAGAATCAAAAAATCCACTTCTAATGGAGATAGAGTACACTTTTAAA
TATCCTTGGAGATGTAGGCTTCCAAGGTTGGAGGCTTGGAACTTTATTCATATAATGAGACAACAA
GATTGCAATATATCACTTGCCAATAACCTTTATAAAATTCCAAAAATATATATGAAAAAGATATTG
GAACTAGCAATACTGGACTTCAATATTTTGCAGTCACAACATCAACATGAAATGAAATTAATATCC
ACATGGTGGAAAAATTCAAGTGCAATTCAATTGGATTTCTTTCGGCATCGTCACATAGAAAGTTAT
TTTTGGTGGGCTAGTCCATTATTTGAACCTGAGTTCAGTACATGTAGAATTAATTGTACCAAATTA
TCTACAAAAATGTTCCTCCTTGACGATATTTATGACACATATGGGACTGTTGAGGAATTGAAACCA
 
TTCACAACAACATTAACAAGATGGGATGTTTCCACAGTTGATAATCATCCAGACTACATGAAAATT
GCTTTCAATTTTTCATATGAGATATATAAGGAAATTGCAAGTGAAGCCGAAAGAAAGCATGGTCCC
TTTGTTTACAAATACCTTCAATCTTGCTGGAAGAGTTATATCGAGGCTTATATGCAAGAAGCAGAA
TGGATAGCTTCTAATCATATACCAGGTTTTGATGAATACTTGATGAATGGAGTAAAAAGTAGCGGC
ATGCGAATTCTAATGATACATGCACTAATACTAATGGATACTCCTTTATCTGATGAAATTTTGGAG
CAACTTGATATCCCATCATCCAAGTCGCAAGCTCTTCTATCATTAATTACTCGACTAGTGGATGAT
GTCAAAGACTTTGAGGATGAACAAGCTCATGGGGAGATGGCATCAAGTATAGAGTGCTACATGAAA
GACAACCATGGTTCTACAAGGGAAGATGCTTTGAATTATCTCAAAATTCGTATAGAGAGTTGTGTG
CAAGAGTTAAATAAGGAGCTTCTCGAGCCTTCAAATATGCATGGATCTTTTAGAAACCTATATCTC
AATGTTGGCATGCGAGTAATATTTTTTATGCTCAATGATGGTGATCTCTTTACACACTCCAATAGA
AAAGAGATACAAGATGCAATAACAAAATTTTTTGTGGAACCAATCATTCCATAG
SEQUENCE ID NO: 8
   ValC
   *Chamaecyparis nootkatensis*
   Amino acid sequence 
MAEMFNGNSSNDGSSCMPVKDALRRTGNHHPNLWTDDFIQSLNSPYSDSSYHKHREILIDEIRDMF
SNGEGDEFGVLENIWFVDVVQRLGIDRHFQEEIKTALDYIYKFWNHDSIFGDLNMVALGFRILRLN
RYVASSDVFKKFKGEEGQFSGFESSDQDAKLEMMLNLYKASELDFPDEDILKEARAFASMYLKHVI
KEYGDIQESKNPLLMEIEYTFKYPWRCRLPRLEAWNFIHIMRQQDCNISLANNLYKIPKIYMKKIL
ELAILDFNILQSQHQHEMKLISTWWKNSSAIQLDFFRHRHIESYFWWASPLFEPEFSTCRINCTKL
STKMFLLDDIYDTYGTVEELKPFTTTLTRWDVSTVDNHPDYMKIAFNFSYEIYKEIASEAERKHGP
FVYKYLQSCWKSYIEAYMQEAEWIASNHIPGFDEYLMNGVKSSGMRILMIHALILMDTPLSDEILE
QLDIPSSKSQALLSLITRLVDDVKDFEDEQAHGEMASSIECYMKDNHGSTREDALNYLKIRIESCV
QELNKELLEPSNMHGSFRNLYLNVGMRVIFFMLNDGDLFTHSNRKEIQDAITKFFVEPIIP
SEQUENCE ID NO: 9
   Valencene synthase
   amino acid sequence 
MAEMFNGNSSNDGSSXMPVKDALRRTGNHHPNLWTDDFIQSLNSPYSDSSYHKHREILIDEIRDMF
SNGEGDEFGVLENIWFVDVVQRLGIDRHFQEEIKTALDYIYKFWNHDSIFGDLNMVALGFRXLRLN
RYVASSDVFKKFKGEEGQFSGFESSDQDAKLEMMLNLYXASELDFPDEDILKEAXAFASMYLKHVI
 
KEYGDIQESKNPLLMEIEYTFKYPWRXRLPRLEAWNFIHIMRQQDXNISLANNLYKIPKIYMKKIL
ELAILDFNILQSQHQHEMKLISTWWKNSSAIQLDFXRXRHIEXYFWWASPLFEPXFSTXRINXTKL
XTKXFLLDDIYDTYGTVEELKPFTTTLTRWDVSTVDNHPDYMKIAFNFSYEIYKEIASEAERKHGP
FXYKYLQSXWKSXXEXYMQEAEWIASNHIPGFDEYLMNGXKXXGMRIXMIHXXXLMDTPLSDEILE
XLDIPSSKSQALLSLITRLVDDVKDXEXEXAHGEMASSIXXYMKXNHGSTREDALNYLKIRIESXV
QELNKELLEPSNMHGSFRNLYLNVGMRXIFXXLNDGDXFXXXNRKEIQDAITKFFVEPIIP
SEQUENCE ID NO: 10
   Valencene synthase
   amino acid sequence 
MAEMFNGNSSNDGSS(CATS)MPVKDALRRTGNHHPNLWTDDFIQSLNSPYSDSSYHKHREILIDE
IRDMFSNGEGDEFGVLENIWFVDVVQRLGIDRHFQEEIKTALDYIYKFWNHDSIFGDLNMVALGFR
(IL)LRLNRYVASSDVFKKFKGEEGQFSGFESSDQDAKLEMMLNLY(KR)ASELDFPDEDILKEA(
RK)AFASMYLKHVIKEYGDIQESKNPLLMEIEYTFKYPWR(CS)RLPRLEAWNFIHIMRQQD(CST
)NISLANNLYKIPKIYMKKILELAILDFNILQSQHQHEMKLISTWWKNSSAIQLDF(FY)R(HD)R
HIE(STA)YFWWASPLFEP(EQ)FST(CA)RIN(CL)TKL(SG)TK(ML)FLLDDIYDTYGTVEEL
KPFTTTLTRWDVSTVDNHPDYMKIAFNFSYEIYKEIASEAERKHGPF(VIMT)YKYLQS(CTV)WK
S(YF)(IFVL)E(AG)YMQEAEWIASNHIPGFDEYLMNG(VLKTW)K(ST)(SGA)GMRI(LIV)M
IH(AS)(LFIY)(ILMV)LMDTPLSDEILE(QESGW)LDIPSSKSQALLSLITRLVDDVKD(FYHS
)E(DNATF)E(QAK)AHGEMASSI(EQ)(CS)YMK(DEQ)NHGSTREDALNYLKIRIES(CTSA)V
QELNKELLEPSNMHGSFRNLYLNVGMR(VT)IF(FHLV)(ML)LNDGD(LSAG)F(TS)(HIV)(S
GAPT)NRKEIQDAITKFFVEPIIP
SEQUENCE ID NO: 11
   >gi|301131133|gb|GU136162.1| Phyla dulcis geraniol synthase (GES) mRNA, complete cds 
ATGGCGAGTGCAAGAAGCACCATATCTTTGTCCTCACAGTCATCTCATCATGGGTTCTCCAAAAAC
TCATTTCCATGGCAACTGAGGCATTCCCGCTTTGTTATGGGTTCTCGAGCACGTACCTGCGCATGC
ATGTCATCATCAGTATCACTGCCTACTGCAACGACGTCGTCCTCAGTCATTACAGGCAACGATGCC
CTCCTCAAATACATACGTCAGCCTATGGTAATTCCTTTGAAAGAAAAGGAGGGCACGAAGAGACGA
GAATATCTGCTGGAGAAAACTGCAAGGGAACTGCAGGGAACTACGGAGGCAGCGGAGAAACTGAAA
TTCATTGATACAATCCAACGGCTGGGAATCTCTTGCTATTTCGAGGATGAAATCAACGGCATACTG
 
CAGGCGGAGTTATCCGATACTGACCAGCTTGAGGACGGCCTCTTCACAACGGCTCTACGCTTCCGT
TTGCTCCGTCACTACGGCTACCAAATCGCTCCCGACGTCTTCCTAAAATTCACGGACCAAAATGGA
AAATTCAAAGAATCCTTAGCGGATGACACACAAGGATTAGTCAGCTTATACGAAGCATCATATATG
GGAGCAAACGGAGAAAACATATTAGAAGAAGCTATGAAATTCACCAAAACTCATCTCCAAGGAAGA
CAACATGCGATGAGAGAAGTGGCTGAAGCCTTGGAGCTTCCGAGGCATCTGAGAATGGCCAGGTTA
GAAGCAAGAAGATACATCGAACAATATGGTACAATGATTGGACATGATAAAGACCTCTTGGAGCTA
GTAATATTGGACTATAACAATGTCCAGGCTCAGCACCAAGCGGAACTCGCCGAAATTGCCAGATGG
TGGAAGGAGCTTGGTCTAGTTGACAAGTTAACTTTCGCGCGAGATAGACCATTGGAGTGCTTTTTG
TGGACTGTCGGTCTTCTACCTGAACCCAAATACTCTGCTTGCCGAATCGAGCTCGCAAAAACAATA
GCCATTCTATTGGTAATCGATGATATCTTCGATACCTATGGGAAAATGGAAGAACTCGCTCTTTTC
ACGGAGGCAATTAGAAGATGGGATCTTGAAGCTATGGAAACCCTTCCCGAGTACATGAAAATATGC
TATATGGCATTGTACAATACCACCAACGAGATATGCTACAAAGTCCTCAAGAAAAATGGATGGAGT
GTTCTCCCATACCTAAGATATACGTGGATGGACATGATAGAAGGTTTTATGGTGGAGGCAAAGTGG
TTCAATGGTGGAAGTGCTCCAAACTTGGAAGAGTACATAGAGAATGGAGTCTCAACGGCTGGGGCA
TACATGGCTTTGGTGCATCTCTTCTTTCTAATTGGGGAAGGTGTCAGTGCGCAAAATGCCCAAATA
TTACTGAAGAAACCCTATCCTAAGCTCTTCTCGGCTGCCGGTCGAATTCTTCGCCTTTGGGATGAT
CTTGGAACGGCTAAGGAGGAGGAAGGAAGAGGTGATCTTGCATCGAGCATACGTTTATTCATGAAA
GAAAAGAACCTAACAACGGAAGAGGAAGGGAGAAATGGTATACAGGAGGAGATATATAGCTTATGG
AAAGACCTAAACGGAGAGCTCATTTCTAAAGGTAGGATGCCATTGGCCATCATCAAAGTGGCACTT
AACATGGCTAGAGCTTCTCAAGTGGTGTACAAGCATGACGAGGACTCTTATTTTTCATGTGTAGAC
AATTATGTGGAGGCCCTGTTCTTCACTCCTCTCCTTTGA
SEQUENCE ID NO: 12
   >gi|301131134|gb|ADK62524.1| geraniol synthase [Phyla dulcis] 
MASARSTISLSSQSSHHGFSKNSFPWQLRHSRFVMGSRARTCACMSSSVSLPTATTSSSVITGNDA
LLKYIRQPMVIPLKEKEGTKRREYLLEKTARELQGTTEAAEKLKFIDTIQRLGISCYFEDEINGIL
QAELSDTDQLEDGLFTTALRFRLLRHYGYQIAPDVFLKFTDQNGKFKESLADDTQGLVSLYEASYM
GANGENILEEAMKFTKTHLQGRQHAMREVAEALELPRHLRMARLEARRYIEQYGTMIGHDKDLLEL
VILDYNNVQAQHQAELAEIARWWKELGLVDKLTFARDRPLECFLWTVGLLPEPKYSACRIELAKTI
AILLVIDDIFDTYGKMEELALFTEAIRRWDLEAMETLPEYMKICYMALYNTTNEICYKVLKKNGWS
VLPYLRYTWMDMIEGFMVEAKWFNGGSAPNLEEYIENGVSTAGAYMALVHLFFLIGEGVSAQNAQI
LLKKPYPKLFSAAGRILRLWDDLGTAKEEEGRGDLASSIRLFMKEKNLTTEEEGRNGIQEEIYSLW
KDLNGELISKGRMPLAIIKVALNMARASQVVYKHDEDSYFSCVDNYVEALFFTPLL
SEQUENCE ID NO: 13
   >gb|DQ234300.1|:1-1812 Perilla frutescens strain 1864 geraniol synthase mRNA, without chloropast targetting 
CGACGCAGTGGAAACTACCAACCTTCTATTTGGGATTTCAACTACGTTCAATCTCTCAACACTCCC
TATAAGGAAGAGAGGTATTTGACAAGGCATGCTGAATTGATTGTGCAAGTGAAACCGTTGCTGGAG
AAAAAAATGGAGGCTGCTCAACAGTTGGAGTTGATTGATGACTTGAACAATCTCGGATTGTCTTAT
TTTTTTCAAGACCGTATTAAGCAGATTTTAAGTTTATATATGACGAGAACCAATGTTTCCACAGT
AATATTAATGATCAAGCAGAGAAAAGGGATTTGTATTTCACAGCTCTTGGATTCAGAATTCTCAGA
CAACATGGTTTTGATGTCTCTCAAGAAGTATTTGATTGTTTCAAGAACGACAGTGGCAGTGATTTT
AAGGCAAGCCTTAGTGACAATACCAAAGGATTGTTACAACTATACGAGGCATCTTTCCTAGTGAGA
GAAGGTGAAGACACACTGGAGCAAGCTAGACAATTCGCCACCAAATTTCTGCGGAGAAAACTTGAT
GAAATTGACGACAATCATCTATTATCATGCATTCACCATTCTTTGGAGATCCCACTTCACTGGAGA
ATTCAAAGGCTGGAGGCAAGATGGTTCTTAGATGCTTACGCGACGAGGCACGACATGAATCCAGTC
ATTCTTGAGCTCGCCAAGCTCGATTTCAATATTATTCAAGCAACACACCAAGAAGAACTCAAGGAT
GTCTCAAGGTGGTGGCAGAATACACGGCTGGCTGAGAAACTCCCATTTGTGAGGGATAGGCTTGTA
GAAAGCTACTTTTGGGCCATTGCGCTGTTTGAGCCTCATCAATATGGATATCAGAGAAGAGTGGCA
GCCAAGATTATTACTCTAGCAACATCTATCGATGATGTTTACGATATCTATGGTACCTTAGATGAA
CTGCAGTTATTTACAGACAACTTTCGAAGATGGGATACTGAATCACTAGGCAGACTTCCATATAGC
ATGCAATTATTTTATATGGTAATCCACAACTTTGTTTCTGAGCTGGCATACGAAATTCTCAAAGAG
AAGGGTTTCATCGTTATCCCATATTTACAGAGATCGTGGGTAGATCTGGCGGAATCATTTTTAAAA
GAAGCAAATTGGTACTACAGTGGATATACACCAAGCCTGGAAGAATATATCGACAACGGCAGCATT
TCAATTGGGGCAGTTGCAGTATTATCCCAAGTTTATTTCACATTAGCAAACTCCATAGAGAAACCT
AAGATCGAGAGCATGTACAAATACCATCACATTCTTCGCCTTTCCGGATTGCTCGTAAGGCTTCAT
GATGATCTAGGAACATCACTGTTTGAGAAGAAGAGAGGCGACGTGCCGAAAGCAGTGGAGATTTGC
ATGAAGGAAAGAAATGTTACCGAGGAAGAGGCGGAAGAACACGTGAAATATCTGATTCGGGAGGCG
TGGAAGGAGATGAACACAGCGACGACGGCAGCCGGTTGTCCGTTTATGGATGAGTTGAATGTGGCC
GCAGCTAATCTCGGAAGAGCGGCGCAGTTTGTGTATCTCGACGGAGATGGTCATGGCGTGCAACAC
TCTAAAATTCATCAACAGATGGGAGGCCTAATGTTCGAGCCATATGTCTGA
SEQUENCE ID NO: 14
   >gi|78192334|gb|ABB30218.1| geraniol synthase [Perilla frutescens] 
RRSGNYQPSIWDFNYVQSLNTPYKEERYLTRHAELIVQVKPLLEKKMEAAQQLELIDDLNNLGLSY
FFQDRIKQILSFIYDENQCFHSNINDQAEKRDLYFTALGFRILRQHGFDVSQEVFDCFKNDSGSDF
KASLSDNTKGLLQLYEASFLVREGEDTLEQARQFATKFLRRKLDEIDDNHLLSCIHHSLEIPLHWR
IQRLEARWFLDAYATRHDMNPVILELAKLDFNIIQATHQEELKDVSRWWQNTRLAEKLPFVRDRLV
ESYFWAIALFEPHQYGYQRRVAAKIITLATSIDDVYDIYGTLDELQLFTDNFRRWDTESLGRLPYS
MQLFYMVIHNFVSELAYEILKEKGFIVIPYLQRSWVDLAESFLKEANWYYSGYTPSLEEYIDNGSI
SIGAVAVLSQVYFTLANSIEKPKIESMYKYHHILRLSGLLVRLHDDLGTSLFEKKRGDVPKAVEIC
MKERNVTEEEAEEHVKYLIREAWKEMNTATTAAGCPFMDELNVAAANLGRAAQFVYLDGDGHGVQH
SKIHQQMGGLMFEPYV
SEQUENCE ID NO: 15
   >gi|38092202:56-1867 Cinnamomum tenuipilum mRNA for geraniol synthase (GerS gene) 
AGAAGATCAGGGAACTACAAGCCCAGCATCTGGGACTATGATTTTGTGCAGTCACTAGGAAGTGGC
TACAAGGTAGAGGCACATGGAACACGTGTGAAGAAGTTGAAGGAAGTTGTAAAGCATTTGTTGAAA
GAAACAGATAGTTCTTTGGCCCAAATAGAACTGATTGACAAACTCCGTCGTCTAGGTCTAAGGTGG
CTCTTCAAAAATGAGATTAAGCAAGTGCTATACACGATATCATCAGACAACACCAGCATAGAAATG
AGGAAAGATCTTCATGCAGTATCAACTCGATTTAGACTTCTTAGACAACATGGGTACAAGGTCTCC
ACAGATGTTTTCAACGACTTCAAAGATGAAAAGGGTTGTTTCAAGCCAAGCCTTTCAATGGACATA
AAGGGAATGTTGAGCTTGTATGAAGCTTCACACCTTGCCTTTCAAGGGGAGACTGTGTTGGATGAG
GCAAGAGCTTTCGTAAGCACACATCTCATGGATATCAAGGAGAACATAGACCCAATCCTTCATAAA
AAAGTAGAGCATGCTTTGGATATGCCTTTGCATTGGAGGTTAGAAAAATTAGAGGCTAGGTGGTAC
ATGGACATATATATGAGGGAAGAAGGCATGAATTCTTCTTTACTTGAATTGGCCATGCTTCATTTC
AACATTGTGCAAACAACATTCCAAACAAATTTAAAGAGTTTGTCAAGGTGGTGGAAAGATTTGGGT
CTTGGAGAGCAGTTGAGCTTCACTAGAGACAGGTTGGTGGAATGTTTCTTTTGGCCGCCGCAATG
ACACCTGAGCCACAATTTGGACGTTGCCAGGAAGTTGTAGCGAAAGTTGCTCAACTCATAATAATA
ATTGACGATATCTATGACGTGTATGGTACGGTGGATGAGCTAGAACTTTTTACTAATGCGATTGAT
AGATGGGATCTTGAGGCAATGGAGCAACTTCCTGAATATATGAAGACCTGTTTCTTAGCTTTATAC
AACAGTATTAATGAAATAGGTTATGACATTTTGAAAGAGGAAGGGCGCAATGTCATACCATACCTT
AGAAATACGTGGACAGAATTGTGTAAAGCATTCTTAGTGGAGGCCAAATGGTATAGTAGTGGATAT
ACACCAACGCTTGAGGAGTATCTGCAAACCTCATGGATTTCGATTGGAAGTCTACCCATGCAAACA
TATGTTTTTGCTCTACTTGGGAAAAATCTAGCACCGGAGAGTAGTGATTTTGCTGAGAAGATCTCG
GATATCTTACGATTGGGAGGAATGATGATTCGACTTCCGGATGATTTGGGAACTTCAACGGATGAA
CTAAAGAGAGGTGATGTTCCAAAATCCATTCAGTGTTACATGCATGAAGCAGGTGTTACAGAGGAT
 
GTTGCTCGCGACCACATAATGGGTCTATTTCAAGAGACATGGAAAAACTCAATGAATACCTTGTG
GAAAGTTCTCTTCCCCATGCCTTTATCGATCATGCTATGAATCTTGGACGTGTCTCCTATTGCACT
TACAAACATGGAGATGGATTTAGTGATGGATTTGGAGATCCTGGCAGTCAAGAGAAAAAGATGTTC
ATGTCTTTATTTGCTGAACCCCTTCAAGTTGATGAAGCCAAGGGTATTTCATTTTATGTTGATGGT
GGATCTGCCTGA
SEQUENCE ID NO: 16
   >gi|38092203|emb|CAD29734.2| geraniol synthase [Cinnamomum tenuipile] 
RRSGNYKPSIWDYDFVQSLGSGYKVEAHGTRVKKLKEVVKHLLKETDSSLAQIELIDKLRRLGLRW
LFKNEIKQVLYTISSDNTSIEMRKDLHAVSTRFRLLRQHGYKVSTDVFNDFKDEKGCFKPSLSMDI
KGMLSLYEASHLAFQGETVLDEARAFVSTHLMDIKENIDPILHKKVEHALDMPLHWRLEKLEARWY
MDIYMREEGMNSSLLELAMLHFNIVQTTFQTNLKSLSRWWKDLGLGEQLSFTRDRLVECFFWAAAM
TPEPQFGRCQEVVAKVAQLIIIIDDIYDVYGTVDELELFTNAIDRWDLEAMEQLPEYMKTCFLALY
NSINEIGYDILKEEGRNVIPYLRNTWTELCKAFLVEAKWYSSGYTPTLEEYLQTSWISIGSLPMQT
YVFALLGKNLAPESSDFAEKISDILRLGGMMIRLPDDLGTSTDELKRGDVPKSIQCYMHEAGVTED
VARDHIMGLFQETWKKLNEYLVESSLPHAFIDHAMNLGRVSYCTYKHGDGFSDGFGDPGSQEKKMF
MSLFAEPLQVDEAKGISFYVDGGSA
SEQUENCE ID NO: 17
   gb|U87908.1|AGU87908:69-1952 Abies grandis myrcene synthase (AG2.2) 
ATGGCTCTGGTTTCTATCTCACCGTTGGCTTCGAAATCTTGCCTGCGCAAGTCGTTGATCAGTTCA
ATTCATGAACATAAGCCTCCCTATAGAACAATCCCAAATCTTGGAATGCGTAGGCGAGGAAATCT
GTCACGCCTTCCATGAGCATCAGTTTGGCCACCGCTGCACCTGATGATGGTGTACAAAGACGCATA
GGTGACTACCATTCCAATATCTGGGACGATGATTTCATACAGTCTCTATCAACGCCTTATGGGGAA
CCCTCTTACCAGGAACGTGCTGAGAGATTAATTGTGGAGGTAAAGAAGATATTCAATTCAATGTAC
CTGGATGATGGAAGATTAATGAGTTCCTTTAATGATCTCATGCAACGCCTTTGGATAGTCGATAGC
GTTGAACGTTTGGGGATAGCTAGACATTTCAAGAACGAGATAACATCAGCTCTGGATTATGTTTTC
CGTTACTGGGAGGAAAACGGCATTGGATGTGGAGAGACAGTATTGTTACTGATCTCAACTCAACT
GCGTTGGGGTTTCGAACTCTTCGATTACACGGGTACACTGTATCTCCAGAGGTTTTAAAAGCTTTT
CAAGATCAAAATGGACAGTTTGTATGCTCCCCCGGTCAGACAGAGGGTGAGATCAGAAGCGTTCTT
AACTTATATCGGGCTTCCCTCATTGCCTTCCCTGGTGAGAAAGTTATGGAAGAAGCTGAAATCTTC
 
TCCACAAGATATTTGAAAGAAGCTCTACAAAAGATTCCAGTCTCCGCTCTTTCACAAGAGATAAAG
TTTGTTATGGAATATGGCTGGCACACAAATTTGCCAAGATTGGAAGCAAGAAATTACATAGACACA
CTTGAGAAAGACACCAGTGCATGGCTCAATAAAAATGCTGGGAAGAAGCTTTTAGAACTTGCAAAA
TTGGAGTTCAATATATTTAACTCCTTACAACAAAAGGAATTACAATATCTTTTGAGATGGTGGAAA
GAGTCGGATTTGCCTAAATTGACATTTGCTCGGCATCGTCATGTGGAATTCTACACTTTGGCCTCT
TGTATTGCCATTGACCCAAAACATTCTGCATTCAGACTAGGCTTCGCCAAAATGTGTCATCTTGTC
ACAGTTTTGGACGATATTTACGACACTTTTGGAACGATTGACGAGCTTGAACTCTTCACATCTGCA
ATTAAGAGATGGAATTCATCAGAGATAGAACACCTTCCAGAATATATGAAATGTGTGTACATGGTC
GTGTTTGAAACTGTAAATGAACTGACACGAGAGGCGGAGAAGACTCAAGGGAGAAACACTCTCAAC
TATGTTCGAAAGGCTTGGGAGGCTTATTTTGATTCATATATGGAAGAAGCAAAATGGATCTCTAAT
GGTTATCTGCCAATGTTTGAAGAGTACCATGAGAATGGGAAAGTGAGCTCTGCATATCGCGTAGCA
ACATTGCAACCCATCCTCACTTTGAATGCATGGCTTCCTGATTACATCTTGAAGGGAATTGATTTT
CCATCCAGGTTCAATGATTTGGCATCGTCCTTCCTTCGGCTACGAGGTGACACACGCTGCTACAAG
GCCGATAGGGATCGTGGTGAAGAAGCTTCGTGTATATCATGTTATATGAAAGACAATCCTGGATCA
ACCGAAGAAGATGCCCTCAATCATATCAATGCCATGGTCAATGACATAATCAAAGAATTAAATTGG
GAACTTCTAAGATCCAACGACAATATTCCAATGCTGGCCAAGAAACATGCTTTTGACATAACAAGA
GCTCTCCACCATCTCTACATATATCGAGATGGCTTTAGTGTTGCCAACAAGGAAACAAAAAAATTG
GTTATGGAAACACTCCTTGAATCTATGCTTTTTTAA
SEQUENCE ID NO: 18
   >gi|2411481|gb|AAB71084.1| myrcene synthase [Abies grandis] 
MALVSISPLASKSCLRKSLISSIHEHKPPYRTIPNLGMRRRGKSVTPSMSISLATAAPDDGVQRRI
GDYHSNIWDDDFIQSLSTPYGEPSYQERAERLIVEVKKIFNSMYLDDGRLMSSFNDLMQRLWIVDS
VERLGIARHFKNEITSALDYVFRYWEENGIGCGRDSIVTDLNSTALGFRTLRLHGYTVSPEVLKAF
QDQNGQFVCSPGQTEGEIRSVLNLYRASLIAFPGEKVMEEAEIFSTRYLKEALQKIPVSALSQEIK
FVMEYGWHTNLPRLEARNYIDTLEKDTSAWLNKNAGKKLLELAKLEFNIFNSLQQKELQYLLRWWK
ESDLPKLTFARHRHVEFYTLASCIAIDPKHSAFRLGFAKMCHLVTVLDDIYDTFGTIDELELFTSA
IKRWNSSEIEHLPEYMKCVYMVVFETVNELTREAEKTQGRNTLNYVRKAWEAYFDSYMEEAKWISN
GYLPMFEEYHENGKVSSAYRVATLQPILTLNAWLPDYILKGIDFPSRFNDLASSFLRLRGDTRCYK
ADRDRGEEASCISCYMKDNPGSTEEDALNHINAMVNDIIKELNWELLRSNDNIPMLAKKHAFDITR
ALHHLYIYRDGFSVANKETKKLVMETLLESMLF
SEQUENCE ID NO: 19
   >gb|AY195609.1|:510-2264 Antirrhinum majus myrcene synthase 1e20 mRNA, complete cds 
ATGATCTATATTTGGATCTGCTTTTATCTCCAAACTACTTTGCTTCCTTGTTCATTGAGTACTCGT
ACCAAATTCGCAATATGTCATAACACGAGTAAACTACATCGTGCTGCATATAAAACTTCTAGATGG
AACATTCCCGGAGATGTCGGATCAACTCCTCCTCCCTCCAAACTTCATCAGGCACTTTGCCTGAAT
GAACACAGTTTAAGTTGCATGGCTGAATTACCAATGGACTACGAAGGAAAAATAAAAGAGACTAGA
CATTTATTACATTTAAAAGGTGAAAATGATCCTATAGAGAGCCTAATTTTTGTGGATGCCACCCTG
AGATTAGGTGTGAACCATCATTTTCAGAAGGAGATCGAAGAAATTCTTCGAAAAAGTTATGCAACG
ATGAAAAGCCCTATTATCTGCGAATACCATACTTTGCACGAAGTTTCACTATTTTTCCGTCTGATG
AGACAACATGGACGCTACGTGTCTGCAGATGTGTTTAACAATTTCAAAGGCGAGAGTGGAGGTTC
AAAGAAGAACTAAAACGAGATACACGAGGTTTAGTGGAGTTATATGAAGCGGCACAACTAAGTTTT
GAAGGAGAACGTATACTTGATGAAGCAGAAAATTTTAGCCGCCAAATTCTCCATGGTAACTTAGCC
GGCATGGAGGATAATTTGCGTAGAAGTGTAGGTAACAAACTAAGGTACCCGTTTCATACGAGCATC
GCAAGATTCACTGGAAGAAACTATGATGATGATCTTGGAGGCATGTACGAATGGGGAAAAACATTA
AGAGAGCTAGCCCTGATGGATTTGCAAGTAGAGCGATCCGTATACCAAGAGGAGTTGCTCCAAGTT
TCCAAGTGGTGGAATGAGCTAGGCTTATATAAGAAGCTAAATCTTGCAAGGAACAGACCATTCGAA
TTTTATACGTGGTCGATGGTTATACTAGCAGATTATATAAACTTGTCAGAGCAGAGAGTGGAGCTC
ACTAAGTCCGTGGCTTTTATTTACTTGATCGATGACATATTTGATGTGTACGGAACACTAGATGAG
CTCATTATTTTTACAGAAGCCGTAAACAAATGGGACTATTCTGCCACTGACACGTTGCCCGAAAAC
ATGAAGATGTGTTGCATGACCCTTCTTGATACAATAAATGGGACTAGCCAAAAAATTTATGAAAAA
CATGGATATAATCCGATTGACTCCCTCAAAACAACTTGGAAAAGTTTGTGCAGTGCATTCCTAGTG
GAGGCTAAATGGTCTGCCTCCGGGAGTCTGCCAAGCGCCAACGAGTATTTGGAGAACGAGAAGGTG
AGCTCAGGAGTGTATGTGGTGCTAGTTCACTTATTTTGTCTTATGGGACTAGGCGGAACTAGCAGA
GGTTCAATCGAGCTAAATGACACACAGGAACTTATGTCCTCTATAGCTATAATTTTTCGTCTTTGG
AATGACTTGGGATCTGCTAAGAATGAGCATCAAAATGGAAAAGATGGATCCTACTTAAATTGCTAC
AAGAAAGAGCATATAAATCTAACAGCTGCACAAGCACATGAGCATGCACTGGAATTGGTAGCAATT
GAATGGAAACGCCTCAATAAAGAATCTTTCAATCTAAATCATGATTCGGTATCTTCTTTCAAGCAA
GCCGCTCTGAATCTTGCAAGGATGGTTCCTCTTATGTATAGCTATGATCACAATCAACGAGGCCCA
GTTCTTGAGGAGTATGTCAAGTTTATGTTGTCGGATTAA
SEQUENCE ID NO: 20
   >gi|30349144|gb|AA041727.1| myrcene synthase 1e20 [Antirrhinum majus]

MIYIWICFYLQTTLLPCSLSTRTKFAICHNTSKLHRAAYKTSRWNIPGDVGSTPPPSKLHQALCLN
EHSLSCMAELPMDYEGKIKETRHLLHLKGENDPIESLIFVDATLRLGVNHHFQKEIEEILRKSYAT
MKSPIICEYHTLHEVSLFFRLMRQHGRYVSADVFNNFKGESGRFKEELKRDTRGLVELYEAAQLSF
EGERILDEAENFSRQILHGNLAGMEDNLRRSVGNKLRYPFHTSIARFTGRNYDDDLGGMYEWGKTL
RELALMDLQVERSVYQEELLQVSKWWNELGLYKKLNLARNRPFEFYTWSMVILADYINLSEQRVEL
TKSVAFIYLIDDIFDVYGTLDELIIFTEAVNKWDYSATDTLPENMKMCCMTLLDTINGTSQKIYEK
HGYNPIDSLKTTWKSLCSAFLVEAKWSASGSLPSANEYLENEKVSSGVYVVLVHLFCLMGLGGTSR
GSIELNDTQELMSSIAIIFRLWNDLGSAKNEHQNGKDGSYLNCYKKEHINLTAAQAHEHALELVAI
EWKRLNKESFNLNHDSVSSFKQAALNLARMVPLMYSYDHNQRGPVLEEYVKFMLSD

## Claims

1. A *Rhodobacter* host cell, comprising a nucleic acid encoding
- enzymes of a mevalonate pathway for making isoprenyl pyrophosphate (IPP) and its isomer dimethylallyl pyrophosphate (DMAPP);
- an enzyme having catalytic activity for the condensation of IPP and DMAPP into geranyl diphosphate (GPP) and
- an enzyme having monoterpene synthase activity in the conversion of GPP into a monoterpene wherein the enzyme having monoterpene synthase activity is a beta-pinene synthase comprising a sequence having at least 40 % sequence identity with SEQ ID NO: 2..

2. *Rhodobacter* host cell according to claim 1, wherein the enzymes of the mevalonate pathway comprise
(i) a heterologous enzyme having catalytic activity in the reaction of acetoacyl-CoA with acetyl-CoA to form HMG-CoA;
(ii) a heterologous enzyme having catalytic activity in the conversion of HMG-CoA to mevalonate;
(iii) a heterologous enzyme having catalytic activity in the phosphorylation of mevalonate to mevalonate 5-phosphate;
(iv) a heterologous enzyme having catalytic activity in the conversion of mevalonate 5-phosphate to mevalonate 5-pyrophosphate;
(v) a heterologous enzyme having catalytic activity in the conversion of mevalonate 5-pyrophosphate to IPP; and
(vi) a heterologous or homologous enzyme having catalytic activity in the reversible conversion of IPP to DMAPP.

3. *Rhodobacter* host cell according to claim 2, wherein the host cell is free of genes encoding a heterologous enzyme having catalytic activity in the reaction of conversion of two molecules of acetyl-CoA to acetoacyl-CoA.

4. *Rhodobacter* host cell according to any one of claims 1 - 3, wherein the beta-pinene synthase comprises a NALI motive and an IGATV motive.

5. *Rhodobacter* host cell according to any one of claims 1 - 4, wherein the beta-pinene synthase comprises a RRXₛW and/or a DDXXD motive, wherein X can be any proteinogenic amino acid and s is an integer preferably in the range of 4-12.

6. *Rhodobacter* host cell according to any of the preceding claims, wherein the enzyme having monoterpene synthase activity comprises a first polypeptide segment and a second polypeptide segment, the first segment comprising a tag-peptide and the second segment comprising a polypeptide having monoterpene synthase activity.

7. A *Rhodobacter* host cell according to any of the preceding claims, wherein the enzymes of the mevalonate pathway comprise
(i) a heterologous enzyme having catalytic activity in the reaction of acetoacyl-CoA with acetyl-CoA to form HMG-CoA;
(ii) a heterologous enzyme having catalytic activity in the conversion of HMG-CoA to mevalonate;
(iii) a heterologous enzyme having catalytic activity in the phosphorylation of mevalonate to mevalonate 5-phosphate;
(iv) a heterologous enzyme having catalytic activity in the conversion of mevalonate 5-phosphate to mevalonate 5-pyrophosphate;
(v) a heterologous enzyme having catalytic activity in the conversion of mevalonate 5-pyrophosphate to IPP; and
(vi) a heterologous or homologous enzyme having catalytic activity in the reversible conversion of IPP to DMAPP.

8. *Rhodobacter* host cell according to any of the preceding claims, wherein the cell is a *Rhodobacter sphaeroides* cell.

9. Use of a *Rhodobacter* host cell according to any of the preceding claims in the production of a monoterpene, preferably a monoterpene selected from the group of beta-pinene, myrcene, alpha-pinene, limonene, sabinene, and geraniol.

10. Method for preparing a monoterpene, comprising culturing a host cell according any of the claims 1 - 8 in a culture medium comprising a carbon source for the monoterpene.

## Patentansprüche

1. *Rhodobacter*-Wirtszelle, umfassend eine Nukleinsäure, die Folgendes codiert:
- Enzyme eines Mevalonatwegs zur Herstellung von Isoprenylpyrophosphat (IPP) und dessen Isomer Dimethylallylpyrophosphat (DMAPP);
- ein Enzym mit katalytischer Aktivität für die Kondensation von IPP und DMAPP zu Geranyldiphosphat (GPP) und
- ein Enzym mit Monoterpen-Synthase-Aktivität bei der Umwandlung von GPP in ein Monoterpen, wobei das Enzym mit Monoterpen-Synthase-Aktivität eine beta-Pinen-Synthase ist, die eine Sequenz mit mindestens 40% Sequenzidentität zu SEQ ID NO: 2 umfasst.

2. *Rhodobacter*-Wirtszelle nach Anspruch 1, wobei die Enzyme des Mevalonatwegs folgende umfassen:
(i) ein heterologes Enzym mit katalytischer Aktivität bei der Reaktion von Acetoacyl-CoA mit Acetyl-CoA unter Bildung von HMG-CoA;
(ii) ein heterologes Enzym mit katalytischer Aktivität bei der Umwandlung von HMG-CoA in Mevalonat;
(iii) ein heterologes Enzym mit katalytischer Aktivität bei der Phosphorylierung von Mevalonat zu Mevalonat-5-phosphat;
(iv) ein heterologes Enzym mit katalytischer Aktivität bei der Umwandlung von Mevalonat-5-phosphat in Mevalonat-5-pyrophosphat;
(v) ein heterologes Enzym mit katalytischer Aktivität bei der Umwandlung von Mevalonat-5-pyrophosphat in IPP und
(vi) ein heterologes oder homologes Enzym mit katalytischer Aktivität bei der reversiblen Umwandlung von IPP in DMAPP.

3. *Rhodobacter*-Wirtszelle nach Anspruch 2, wobei die Wirtszelle frei von Genen ist, die ein heterologes Enzym mit katalytischer Aktivität bei der Umwandlungsreaktion von zwei Molekülen Acetyl-CoA in Acetoacyl-CoA codieren.

4. *Rhodobacter*-Wirtszelle nach einem der Ansprüche 1-3, wobei die beta-Pinen-Synthase ein NALI-Motiv und ein IGATV-Motiv umfasst.

5. *Rhodobacter*-Wirtszelle nach einem der Ansprüche 1-4, wobei die beta-Pinen-Synthase ein RRXₛW- und/oder DDXXD-Motiv umfasst, wobei X eine beliebige proteinogene Aminosäure sein kann und s ist eine ganze Zahl, vorzugsweise im Bereich von 4-12, ist.

6. *Rhodobacter*-Wirtszelle nach einem der vorhergehenden Ansprüche, wobei das Enzym mit Monoterpen-Synthase-Aktivität ein erstes Polypeptidsegment und ein zweites Polypeptidsegment umfasst, wobei das erste Segment ein Tag-Peptid umfasst und das zweite Segment ein Polypeptid mit Monoterpen-Synthase-Aktivität umfasst.

7. *Rhodobacter*-Wirtszelle nach einem der vorhergehenden Ansprüche, wobei die Enzyme des Mevalonatwegs folgende umfassen:
(i) ein heterologes Enzym mit katalytischer Aktivität bei der Reaktion von Acetoacyl-CoA mit Acetyl-CoA unter Bildung von HMG-CoA;
(ii) ein heterologes Enzym mit katalytischer Aktivität bei der Umwandlung von HMG-CoA in Mevalonat;
(iii) ein heterologes Enzym mit katalytischer Aktivität bei der Phosphorylierung von Mevalonat zu Mevalonat-5-phosphat;
(iv) ein heterologes Enzym mit katalytischer Aktivität bei der Umwandlung von Mevalonat-5-phosphat in Mevalonat-5-pyrophosphat;
(v) ein heterologes Enzym mit katalytischer Aktivität bei der Umwandlung von Mevalonat-5-pyrophosphat in IPP und
(vi) ein heterologes oder homologes Enzym mit katalytischer Aktivität bei der reversiblen Umwandlung von IPP in DMAPP.

8. *Rhodobacter*-Wirtszelle nach einem der vorhergehenden Ansprüche, wobei es sich bei der Zelle um eine *Rhodobacter sphaeroides-*Zelle handelt.

9. Verwendung einer *Rhodobacter*-Wirtszelle nach einem der vorhergehenden Ansprüche bei der Produktion eines Monoterpens, vorzugsweise eines aus der Gruppe mit beta-Pinen, Myrcen, alpha-Pinen, Limonen, Sabinen und Geraniol ausgewählten Monoterpens.

10. Verfahren zur Herstellung eines Monoterpens, umfassend das Züchten einer Wirtszelle nach einem der Ansprüche 1-8 in einem Kulturmedium, das eine Kohlenstoffquelle für das Monoterpen umfasst.

## Revendications

1. Cellule hôte de *Rhodobacter,* comprenant un acide nucléique codant pour
- des enzymes d'une voie du mévalonate afin de préparer l'isoprényl-pyrophosphate (IPP) et son isomère le diméthylallyl-pyrophosphate (DMAPP) ;
- une enzyme ayant une activité catalytique pour la condensation de l'IPP et du DMAPP en géranyl-diphosphate (GPP) ; et
- une enzyme ayant une activité de monoterpène synthase dans la conversion du GPP en monoterpène, où l'enzyme ayant une activité de monoterpène synthase est une bêta-pinène synthase comprenant une séquence ayant au moins 40% d'identité de séquence avec SEQ ID n° 2.

2. Cellule hôte de *Rhodobacter* selon la revendication 1, où les enzymes de la voie du mévalonate comprennent :
(i) une enzyme hétérologue ayant une activité catalytique dans la réaction de l'acétoacyl-CoA avec de l'acétyl-CoA afin de former du HMG-CoA ;
(ii) une enzyme hétérologue ayant une activité catalytique dans la conversion du HMG-CoA en mévalonate ;
(iii) une enzyme hétérologue ayant une activité catalytique dans la phosphorylation du mévalonate en mévalonate-5-phosphate ;
(iv) une enzyme hétérologue ayant une activité catalytique dans la conversion du mévalonate-5-phosphate en mévalonate-5-pyrophosphate ;
(v) une enzyme hétérologue ayant une activité catalytique dans la conversion du mévalonate-5-pyrophosphate en IPP ; et
(vi) une enzyme hétérologue ou homologue ayant une activité catalytique dans la conversion réversible de l'IPP en DMAPP.

3. Cellule hôte de *Rhodobacter* selon la revendication 2, où la cellule hôte est dépourvue de gènes codant pour une enzyme hétérologue ayant une activité catalytique dans la réaction de conversion de deux molécules d'acétyl-CoA en acétoacyl-CoA.

4. Cellule hôte de *Rhodobacter* selon l'une quelconque des revendications 1-3, où la bêta-pinène synthase comprend un motif NALI et un motif IGATV.

5. Cellule hôte de *Rhodobacter* selon l'une quelconque des revendications 1-4, où la bêta-pinène synthase comprend un motif RRXₛW et/ou DDXXD, où X peut être un acide aminé protéinogène quelconque et s est un nombre entier se trouvant de préférence dans la plage de 4-12.

6. Cellule hôte de *Rhodobacter* selon l'une quelconque des revendications précédentes, où l'enzyme ayant une activité de monoterpène synthase comprend un premier segment de polypeptide et un deuxième segment de polypeptide, le premier segment comprenant un peptide étiquette et le deuxième segment comprenant un polypeptide ayant une activité de monoterpène synthase.

7. Cellule hôte de *Rhodobacter* selon l'une quelconque des revendications précédentes, où les enzymes de la voie du mévalonate comprennent :
(i) une enzyme hétérologue ayant une activité catalytique dans la réaction de l'acétoacyl-CoA avec de l'acétyl-CoA afin de former du HMG-CoA ;
(ii) une enzyme hétérologue ayant une activité catalytique dans la conversion du HMG-CoA en mévalonate ;
(iii) une enzyme hétérologue ayant une activité catalytique dans la phosphorylation du mévalonate en mévalonate-5-phosphate ;
(iv) une enzyme hétérologue ayant une activité catalytique dans la conversion du mévalonate-5-phosphate en mévalonate-5-pyrophosphate ;
(v) une enzyme hétérologue ayant une activité catalytique dans la conversion du mévalonate-5-pyrophosphate en IPP ; et
(vi) une enzyme hétérologue ou homologue ayant une activité catalytique dans la conversion réversible de l'IPP en DMAPP.

8. Cellule hôte de *Rhodobacter* selon l'une quelconque des revendications précédentes, où la cellule est une cellule de *Rhodobacter sphaeroides.*

9. Utilisation d'une cellule hôte de *Rhodobacter* selon l'une quelconque des revendications précédentes, dans la production d'un monoterpène, préférablement un monoterpène choisi dans le groupe constitué par le bêta-pinène, le myrcène, l'alpha-pinène, le limonène, le sabinène, et le géraniol.

10. Méthode de préparation d'un monoterpène, comprenant la culture d'une cellule hôte selon l'une quelconque des revendications 1-8 dans un milieu de culture comprenant une source de carbone pour le monoterpène.
